# EUROPEAN PATENT SPECIFICATION

(11) **EP 3 208 333 B1**
(45) Date of publication and mention of the grant of the patent: **16.12.2020**
(21) Application number: 15850481.1
(22) Date of filing: 14.10.2015
(51) Int. Cl.: C12N 15/10, C12Q 1/6809

(54) **METHOD FOR DETECTING AND QUANTIFYING TARGET NUCLEIC ACID IN TEST SAMPLE USING NOVEL POSITIVE CONTROL NUCLEIC ACID**
VERFAHREN ZUM NACHWEIS UND ZUR QUANTIFIZIERUNG VON ZIELNUKLEINSÄUREN IN EINER TESTPROBE MIT EINER NEUARTIGEN POSITIVE-CONTROL-NUKLEINSÄURE
PROCÉDÉ DE DÉTECTION ET DE QUANTIFICATION D'ACIDE NUCLÉIQUE CIBLE DANS UN ÉCHANTILLON POUR ESSAI UTILISANT LE NOUVEL ACIDE NUCLÉIQUE DE CONTRÔLE POSITIF

(30) Priority: 17.10.2014 JP 2014212496
(43) Date of publication of application: 23.08.2017
(73) Proprietor: National University Corporation Tokyo Medical and Dental University, Bunkyo-ku Tokyo 113-8510 (JP)
(72) Inventor: SHIMIZU, Norio, Tokyo 113-8510 (JP); WATANABE, Ken, Tokyo 113-8510 (JP); TOMARU, Yasuhiro, Ushiku-shi Ibaraki 300-1234 (JP)
(74) Representative: Gill, Siân Victoria
(86) International application number: PCT/JP2015/005198
(87) International publication number: WO 2016/059798

(56) References cited:
- EP-A1- 2 660 332
- WO-A1-2005/005659
- WO-A1-2010/111509
- WO-A2-2014/063051
- JP-A- 2004 329 209
- JP-A- 2004 507 248
- JP-A- 2007 130 006
- US-A1- 2007 141 563
- BEHETS J ET AL: "Development and evaluation of a Taqman duplex real-time PCR quantification method for reliable enumeration of Legionella pneumophila in water samples", JOURNAL OF MICROBIOLOGICAL METHODS, ELSEVIER, AMSTERDAM, NL, vol. 68, no. 1, 1 January 2007 (2007-01-01), pages 137-144, XP022731911, ISSN: 0167-7012, DOI: 10.1016/J.MIMET.2006.07.002 [retrieved on 2007-01-10]
- SATOKO NAKANO ET AL: "Establishment of Multiplex Solid-Phase Strip PCR Test for Detection of 24 Ocular Infectious Disease Pathogens", INVESTIGATIVE OPTHALMOLOGY & VISUAL SCIENCE, vol. 58, no. 3, 10 March 2017 (2017-03-10) , page 1553, XP055450489, US ISSN: 1552-5783, DOI: 10.1167/iovs.16-20556

## Description

### Technical Field

The present invention relates to a method for detecting or quantifying a target nucleic acid in a test sample. More specifically, the present invention relates to a method for detecting or quantifying a target nucleic acid in a test sample while confirming the presence or absence of contamination of a container for the test sample with a positive control nucleic acid.

### Background Art

Techniques related to the amplification and detection of nucleic acids are widely utilized in various fields such as the diagnosis of infectious diseases, nucleic acid testing for agricultural crops, and gene diagnosis. Various methods are used as methods for performing the amplification or detection of nucleic acids.

In addition to typical PCR (polymerase chain reaction), for example, TRC (transcription-reverse transcription concerted reaction), TMA (transcription-mediated amplification), NASBA (nucleic acid sequence-based amplification), LAMP (loop-mediated isothermal amplification), SMAP (smart amplification process), and ICAN (isothermal and chimeric primer-initiated amplification of nucleic acids) are known as nucleic acid amplification methods. Also, intercalator and probe methods are typically known as methods for detecting a target amplification product from amplification products. The probe method is often used because of its more highly sensitive and highly accurate detection. Many types of probes are used in the probe method, and, for example, hybridization-probes, TaqMan(R) probes, Q probes, cycling probes, Eprobe(R), Qprobe, and molecular beacon probes are known.

The amplification and detection of nucleic acids may produce false negative, i.e., negative results for some cause in spite of being positive in actuality. In order to avoid such false negative, the detection or quantification of a target nucleic acid in a test sample is often carried out by performing similar nucleic acid amplification or detection operation using a positive control nucleic acid instead of the test sample in a reaction container different from a reaction container for the test sample. If positive results are obtained using the positive control nucleic acid, negative results obtained using the test sample are highly reliable, and the possibility of false negative can be drastically reduced. Also, in order to more accurately measure the copy number or concentration of a target nucleic acid in a test sample, similar nucleic acid amplification or detection operation is often performed using a known concentration of a positive control nucleic acid in a reaction container different from a reaction container for the test sample. For example, patent document 1 describes a method for detecting a target polynucleotide, the method using an external control polynucleotide (ECP) as a positive control nucleic acid.

The positive control nucleic acid inevitably has a primer-binding sequence and a probe-binding sequence, as with the target nucleic acid, in order to exert functions as a positive control. Thus, in the case of using a positive control nucleic acid in combination with a test sample in nucleic acid amplification and detection, the unintended contamination of a reaction container for the test sample with the positive control nucleic acid or its amplification product is disadvantageously responsible for false-positive reaction. Particularly, in nucleic acid amplification and detection tests, unlike other general comparative tests, it is very highly possible that contamination with even a trace amount of a positive control nucleic acid causes false positive because an added positive control is amplified hundreds of thousands of or millions of times. In recent years, a large number of reactions have been efficiently performed at once in many cases using an instrument in which many reaction containers are located in proximity to each other, such as a strip tube composed of a plurality of tubes connected in a line or a microplate having many wells arranged on a plate. Therefore, there is still great concern about the unintended contamination of a reaction container for the test sample with the positive control nucleic acid. In addition, in recent years, reagents necessary for nucleic acid amplification reaction, such as positive control nucleic acids and primer sets, have been arranged or immobilized in their respective reaction containers in many cases in the aforementioned instrument in which many reaction containers are located in proximity to each other, so as to attain more convenient and rapid amplification and detection of nucleic acids. When positive control nucleic acids are arranged or immobilized in a reaction container, the unintended contamination of a container for the test sample with even a very small amount of a positive control nucleic acid is responsible for false positive or responsible for a large deviation of the quantification value of the target nucleic acid because higher concentrations of positive control nucleic acids are handled.

A general problem associated with the conventional amplification and detection of nucleic acids is carryover contamination. The carryover contamination refers to the occurrence of false positive or the like resulting from the contamination of a container for newly performed nucleic acid amplification reaction with an amplification product of preceding nucleic acid amplification reaction. An approach which involves performing PCR using a substrate containing dUTP instead of dTTP, incorporating an uracil base into the amplification product, and degrading a contaminating amplification product by treatment with uracil-N-glycosylase (UNG) upon next PCR (dUTP/UDG contamination removal method) is well-known as a method for preventing such carryover contamination (non-patent document 1). Also, a method further using polyamine promoting the degradation of abasic DNA is disclosed as a modification of this dUTP/UDG contamination removal method (patent document 2).

However, there has been no previous report about a practical method for preventing the contamination of, particularly, a container for a test sample with a positive control nucleic acid in nucleic acid amplification and detection, or a practical method for detecting or quantifying a target nucleic acid in a test sample while conveniently and rapidly confirming the presence or absence of contamination thereof.

### Prior Art Documents

### Patent Documents

Patent Document 1: Japanese unexamined Patent Application Publication (Translation of PCT Application) No. 2004-507248 discloses methods of nucleic acid amplification with external controls that verify the absence or presence of specific target sequences, and correct primers and probes. A single-stranded, external control polynucleotide is amplified with primers of the same sequence as target primers. Probes with detectable labels and sequences specific for target and external control polynucleotides allow for detection and measurement. The primers and the detectable probe are adjacent or substantially adjacent when hybridization of a self-quenching probe complementary to both target and external control polynucleotides.
Patent Document 2: Japanese unexamined Patent Application Publication No. 2010-4884

WO 2010/111509 discloses methods and kits incorporating a discriminating positive control for determining whether a particular microorganism or group of microorganisms are present in a sample.

WO 2014/063051 discloses components and methods for polymerase chain reaction assays that minimize both handling of material and time spent running samples.

WO 2005/005659 discloses a method for the identification of the diarrheagenic E. coli groups. The method includes an internal positive PCR control and the carry-over prevention system, UNG, which makes it ideal for routine diagnostic analyses.

US 2007/141563 discloses a method for detecting a target biomolecule in test sample by adding an internal control biomolecule to the test sample; to a negative control sample, to a positive control sample and to a reagent control sample or adding an internal control biomolecule to the test sample, to a negative control sample, to a positive control sample comprising the target biomolecule and providing a reagent control sample comprising the target biomolecule, determining in each sample a signal, and verifying the signal thereby detecting the target biomolecule.

EP 2660332 discloses detecting or quantitating at least two different target nucleic acids using a single positive control stock solution comprising a mixture of positive control nucleic acids for the different target nucleic acids to be detected or quantitated.

### Non-patent Document

Non-patent Document: Gene, Vol. 93 (1), 125-128 (1990)

Behets, et al in Journal of Microbiological Methods Volume 68, Issue 1, January 2007, pages 137-144 disclose the development and evaluation of a specific *Legionella pneumophila* Taqman duplex real-time PCR (qPCR) for fast and reliable quantification of this human pathogen in suspected man-made water systems.

### Summary of the Invention

### Object to be Solved by the Invention

An object of the present invention is to provide a set capable of conveniently and rapidly confirming the presence or absence of contamination of a container for a test sample, the set comprising a positive control nucleic acid and a probe specific for the positive control nucleic acid. Another object of the present invention is to provide a method for detecting or quantifying a target nucleic acid in a test sample while conveniently and rapidly confirming the presence or absence of contamination of a container for the test sample with a positive control nucleic acid.

### Means to Solve the Object

The present inventors have conducted diligent studies to attain the objects and consequently completed the present invention by finding that a positive control nucleic acid is any nucleic acid selected from (I) a single-stranded nucleic acid consisting of a nucleotide sequence having features (A) to (D) mentioned later, (II) a single-stranded nucleic acid consisting of a nucleotide sequence complementary to the nucleotide sequence of the single-stranded nucleic acid (I), and (III) a double-stranded nucleic acid formed from the single-stranded nucleic acid (I) and the single-stranded nucleic acid (II),
wherein the positive control nucleic acid can be amplified using the primer set specific for the target nucleic acid,
wherein the nucleotide sequence of a probe specific for the positive control nucleic acid is a nucleotide sequence that has 70% or lower sequence identity to the sequence of any portion of the genomes of an organism species from which the test sample is derived and an organism species from which the target nucleic acid is derived, and transcripts thereof, and has a Tm value of 65°C or lower; and
wherein the nucleotide sequence of the probe specific for the positive control nucleic acid is a nucleotide sequence that has 70% or lower sequence identity to the sequence of any portion of the genomes of 20 or more species selected from the group consisting of mammals, bacteria, fungi and viruses, and transcripts thereof, and has a Tm value of 65°C or lower:
(A) comprising nucleotide sequence A1 of a 5' primer specific for the target nucleic acid and nucleotide sequence A2 complementary to a 3' primer specific for the target nucleic acid;
(B) comprising nucleotide sequence B1 complementary to the probe specific for the target nucleic acid, or nucleotide sequence B2 complementary to the nucleotide sequence B1;
(C) nucleotide sequence C1 complementary to the probe specific for the positive control nucleic acid, or nucleotide sequence C2 complementary to the nucleotide sequence C1; and
(D) the nucleotide sequence B1 or B2 and the nucleotide sequence C1 or C2 are positioned between the nucleotide sequences A1 and A2.

Specifically, the present invention relates to:
(1) a set for use for at least confirming the presence or absence of contamination of a container for a test sample with a positive control nucleic acid in the detection or quantification of a target nucleic acid in a test sample using a primer set specific for the target nucleic acid and a probe specific for the target nucleic acid, the set comprising a positive control nucleic acid and a probe specific for the positive control nucleic acid,
   wherein the positive control nucleic acid serves as a positive control of the target nucleic acid and is any one of a nucleic acid selected from (I) a single-stranded nucleic acid consisting of a nucleotide sequence having the following features (A) to (D), (II) a single-stranded nucleic acid consisting of a nucleotide sequence complementary to the nucleotide sequence of the single-stranded nucleic acid (I), and (III) a double-stranded nucleic acid formed from the single-stranded nucleic acid (I) and the single-stranded nucleic acid (II),
   wherein the positive control nucleic acid can be amplified using the primer set specific for the target nucleic acid,
   wherein the nucleotide sequence of the probe specific for the positive control nucleic acid is a nucleotide sequence that has 70% or lower sequence identity to the sequence of any portion of the genomes of an organism species from which the test sample is derived and an organism species from which the target nucleic acid is derived, and transcripts thereof, and has a Tm value of 65°C or lower; and
   wherein the nucleotide sequence of the probe specific for the positive control nucleic acid is a nucleotide sequence that has 70% or lower sequence identity to the sequence of any portion of the genomes of 20 or more species selected from the group consisting of mammals, bacteria, fungi and viruses, and transcripts thereof, and has a Tm value of 65°C or lower:
   (A) comprising a nucleotide sequence A1 of a 5' primer specific for the target nucleic acid and a nucleotide sequence A2 complementary to a 3' primer specific for the target nucleic acid;
   (B) comprising a nucleotide sequence B1 complementary to the probe specific for the target nucleic acid, or a nucleotide sequence B2 complementary to the nucleotide sequence B1;
   (C) comprising a nucleotide sequence C1 complementary to the probe specific for the positive control nucleic acid, or a nucleotide sequence C2 complementary to the nucleotide sequence C1; and
   (D) the nucleotide sequence B1 or B2 and the nucleotide sequence C1 or C2 are positioned between the nucleotide sequences A1 and A2;
(2) the set according to (1), wherein the nucleotide sequence (I) in the nucleotide sequence of the positive control nucleic acid is
   (I') a nucleotide sequence wherein the nucleotide sequence C1 or C2 is inserted to a portion other than the nucleotide sequences A1 and A2 and the nucleotide sequence B1 or B2 in a single-stranded nucleic acid having the nucleotide sequences A1 and A2 and the nucleotide sequence B1 or B2 in a nucleotide sequence derived from the nucleotide sequence of the target nucleic acid, or
   a nucleotide sequence wherein one or more nucleotide sequence(s) of a portion other than the nucleotide sequences A1 and A2 and the nucleotide sequence B1 or B2 is substituted with the nucleotide sequence C1 or C2 in a single-stranded nucleic acid having the nucleotide sequences A1 and A2 and the nucleotide sequence B1 or B2 in a nucleotide sequence derived from the nucleotide sequence of the target nucleic acid,
(3) the set according to (1) or (2), wherein the number of nucleotides of the nucleotide sequences A1 and A2 is within the range of 15 to 50, respectively, the number of nucleotides of the nucleotide sequences B1 and B2 is within the range of 15 to 100, respectively, the number of nucleotides of the nucleotide sequences C1 and C2 is within the range of 15 to 100, respectively, and the number of nucleotides of the probe specific for the positive control nucleic acid is within the range of 15 to 100, and
(4) the set according to any one of (1) to (3), wherein the nucleotide sequence of the probe specific for the positive control nucleic acid is a nucleotide sequence having a linkage of two or more nucleotide sequences, which is selected from the group consisting of repeat nucleotide sequences having 4 to 8 nucleotides obtained by repeating twice to four times any of the following nucleotide sequences having 2 nucleotides:
   AT, AG, AC, TA, TG, TC, GA, GT, GC, CA, CT, and CG.

The present invention also relates to:
(5) a kit for use in the detection or quantification of a target nucleic acid in a test sample, the kit comprising: a primer set specific for the target nucleic acid; a probe specific for the target nucleic acid; a set according to any one of (1) to (4) comprising a positive control nucleic acid and a probe specific for the positive control nucleic acid; and two or more reaction containers, wherein
   the primer set specific for the target nucleic acid, the probe specific for the target nucleic acid, and the probe specific for the positive control nucleic acid are immobilized in at least one of the reaction container(s) for the test sample among the two or more reaction containers, and the primer set specific for the target nucleic acid, the probe specific for the target nucleic acid, the positive control nucleic acid, and the probe specific for the positive control nucleic acid are immobilized in at least one of the remaining reaction container(s) for the positive control nucleic acid,
(6) the kit according to (5) for use in the detection or quantification of two different types of target nucleic acids in a test sample, wherein
   the kit comprises: two types of primer sets each specific for the two types of target nucleic acids; two types of probes each specific for the two types of target nucleic acids; a set comprising two types of positive control nucleic acids each serving as a positive control for the two types of target nucleic acids, and one or two types of probes specific for the two types of positive control nucleic acids; and two or more reaction containers, wherein
   among the two types of target nucleic acids, one type of target nucleic acid is a nucleic acid of a gene other than housekeeping gene, and the other type of target nucleic acid is a nucleic acid of a housekeeping gene,
   the two types of primer sets, the two types of probes, and the one or two types of probes are immobilized in each individual reaction container for the test sample, and the two types of primer sets, the two types of probes, the two types of positive control nucleic acids, and the one or two types of probes are immobilized in each of the reaction container for the positive control nucleic acids,
(7) the kit according to (5) or (6), wherein polymerase is further immobilized in each reaction container,
(8) the kit according to (6) or (7), wherein
   the two or more reaction containers are three or more reaction containers connected to each other,
   among the reaction containers, one or more reaction container(s) is a reaction container for the test sample, one or more reaction container(s) is a reaction container for the positive control nucleic acids, and one or more reaction container(s) is a reaction container for a negative control, and
   wherein the two types of primer sets each specific for the two types of target nucleic acids, the two types of probes each specific for the two types of target nucleic acids, and one or two types of probes specific for the two types of positive control nucleic acids are immobilized in the reaction container for a negative control, and
(9) the kit according to (8), wherein
   the three or more reaction containers connected to each other are four or more reaction containers connected to each other,
   two or more reaction containers are reaction containers for the positive control nucleic acids among the reaction containers,
   the amount of the positive control nucleic acid immobilized in the two or more reaction containers gradually differs, and the amount of the positive control nucleic acid in a reaction container having the largest amount of the positive control nucleic acid among the two or more reaction containers is 10 or more times the amount of the positive control nucleic acid in a reaction container having the smallest amount of the positive control nucleic acid.

The present invention also relates to:
(10) a method for detecting or quantifying a target nucleic acid in a test sample while confirming the presence or absence of contamination of a container for the test sample with a positive control nucleic acid, the method comprising:
   (a) step "a" of performing a nucleic acid amplification reaction using a nucleic acid obtained from the test sample and a primer set specific for the target nucleic acid in a reaction container for the test sample to obtain an amplification product;
   (b) step "b" of performing a nucleic acid amplification reaction using the positive control nucleic acid and the primer set specific for the target nucleic acid in a reaction container for the positive control nucleic acid to obtain an amplification product;
   (c) step "c" of contacting the amplification product obtained in the step "a" with a probe specific for the target nucleic acid and a probe specific for the positive control nucleic acid in the reaction container for the test sample, and detecting or quantifying the presence or absence of the target nucleic acid and the positive control nucleic acid; and
   (d) step "d" of contacting the amplification product obtained in the step "b" with the probe specific for the target nucleic acid and the probe specific for the positive control nucleic acid in the reaction container for the positive control nucleic acid, and detecting or quantifying the presence or absence of the target nucleic acid and the positive control nucleic acid, wherein
   when the positive control nucleic acid is not detected in the step "c", it can be confirmed that the container for the test sample is not contaminated with the positive control nucleic acid,
   the positive control nucleic acid serves as a positive control of the target nucleic acid and is any one of a nucleic acid selected from (I) a single-stranded nucleic acid consisting of a nucleotide sequence having the following features (A) to (D), (II) a single-stranded nucleic acid consisting of a nucleotide sequence complementary to the nucleotide sequence of the single-stranded nucleic acid (I), and (III) a double-stranded nucleic acid formed from the single-stranded nucleic acid (I) and the single-stranded nucleic acid (II),
   wherein the positive control nucleic acid can be amplified using the primer set specific for the target nucleic acid,
   wherein the nucleotide sequence of the probe specific for the positive control nucleic acid is a nucleotide sequence that has 70% or lower sequence identity to the sequence of any portion of the genomes of an organism species from which the test sample is derived and an organism species from which the target nucleic acid is derived, and transcripts thereof, and has a Tm value of 65°C or lower; and
   wherein the nucleotide sequence of the probe specific for the positive control nucleic acid is a nucleotide sequence that has 70% or lower sequence identity to the sequence of any portion of the genomes of 20 or more species selected from the group consisting of mammals, bacteria, fungi and viruses, and transcripts thereof, and has a Tm value of 65°C or lower:
   (A) comprising a nucleotide sequence A1 of a 5' primer specific for the target nucleic acid and a nucleotide sequence A2 complementary to a 3' primer specific for the target nucleic acid;
   (B) comprising a nucleotide sequence B1 complementary to the probe specific for the target nucleic acid, or a nucleotide sequence B2 complementary to the nucleotide sequence B1;
   (C) comprising a nucleotide sequence C1 complementary to the probe specific for the positive control nucleic acid, or a nucleotide sequence C2 complementary to the nucleotide sequence C1; and
   (D) the nucleotide sequence B1 or B2 and the nucleotide sequence C1 or C2 are positioned between the nucleotide sequences A1 and A2, and
(11) the method according to (10) for detecting or quantifying two different types of target nucleic acids in a test sample while confirming the presence or absence of contamination of a container for the test sample with a positive control nucleic acid, the method comprising
   using two types of primer sets each specific for the two types of target nucleic acids as the primer set specific for the target nucleic acid,
   using two types of positive control nucleic acids as the positive control nucleic acid,
   using two types of probes each specific for the two types of target nucleic acids as the probe specific for the target nucleic acid,
   using one or two types of probes specific for the two types of positive control nucleic acids as the probe specific for the positive control nucleic acid, and
   detecting or quantifying the presence or absence of the two types of target nucleic acids and the two types of positive control nucleic acids, wherein
   when the positive control nucleic acid is not detected in the step "c", it can be confirmed that the container for the test sample is not contaminated with the positive control nucleic acid, and
   among the two types of target nucleic acids, one type of target nucleic acid is a nucleic acid of a gene other than housekeeping gene, and the other type of target nucleic acid is a nucleic acid of a housekeeping gene.

### Effect of the Invention

The present invention can provide a set capable of conveniently and rapidly confirming the presence or absence of contamination of a container for a test sample, the set comprising a positive control nucleic acid and a probe specific for the positive control nucleic acid. The present invention can also provide a method for detecting or quantifying a target nucleic acid in a test sample while conveniently and rapidly confirming the presence or absence of contamination of a container for the test sample with a positive control nucleic acid.

### Brief Description of Drawings

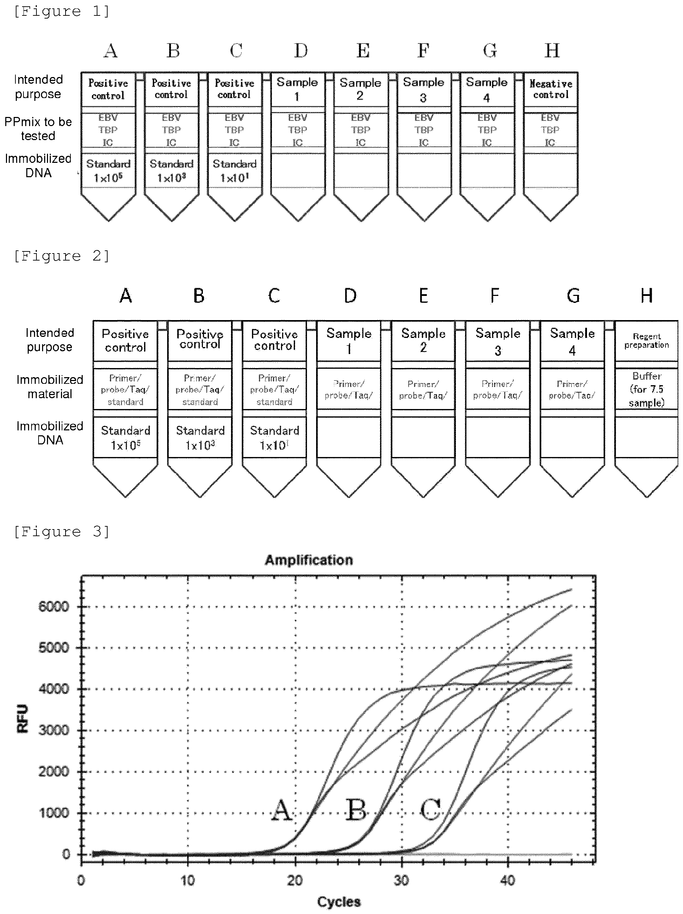

[Figure 1] Figure 1 is a diagram showing a reagent-immobilized 8-strip PCR tube, which is one embodiment of the kit of the present invention. A to C each denote a reaction container for positive control nucleic acids, and D to G each denote a reaction container for a test sample. H denotes a reaction container for a negative control.
[Figure 2] Figure 2 is a diagram showing a reagent-immobilized 8-strip PCR tube, which is one embodiment of the kit of the present invention. A to C each denote a reaction container for positive control nucleic acids. D to G each denote a reaction container for a test sample. H denotes a reaction container for a buffer for nucleic acid amplification reaction.
[Figure 3] Figure 3 is a diagram showing an amplification curve of real-time PCR assay in reaction containers A to C (reaction containers for positive control nucleic acids) in Example 4 of the present application. The abscissa depicts the number of reaction cycles, and the ordinate depicts fluorescence intensity RFU.

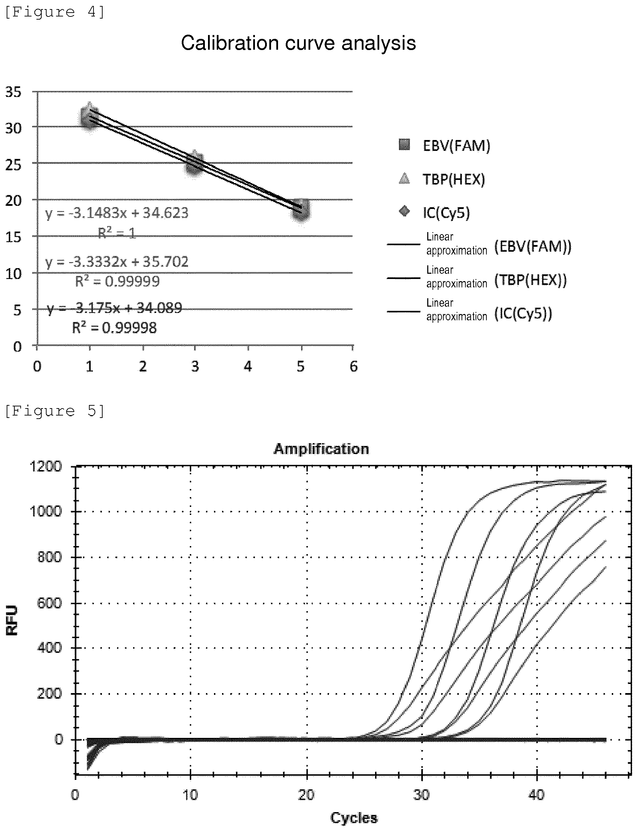

[Figure 4] Figure 4 is a diagram showing a regression equation (calibration curve) determined by regression analysis using a Cq value calculated about each target (EBV, TBP, and IC) on the basis of the results of Figure 3 (Y) and a numerical value of the copy number of each positive control nucleic acid indicated in common logarithm (X). The abscissa depicts the numerical value of the copy number of each positive control nucleic acid indicated in common logarithm, and the ordinate depicts the Cq value.
[Figure 5] Figure 5 is a diagram showing an amplification curve of real-time PCR assay in reaction containers D to G (reaction containers for a test sample) in Example 4 of the present application. The abscissa depicts the number of reaction cycles, and the ordinate depicts fluorescence intensity RFU.

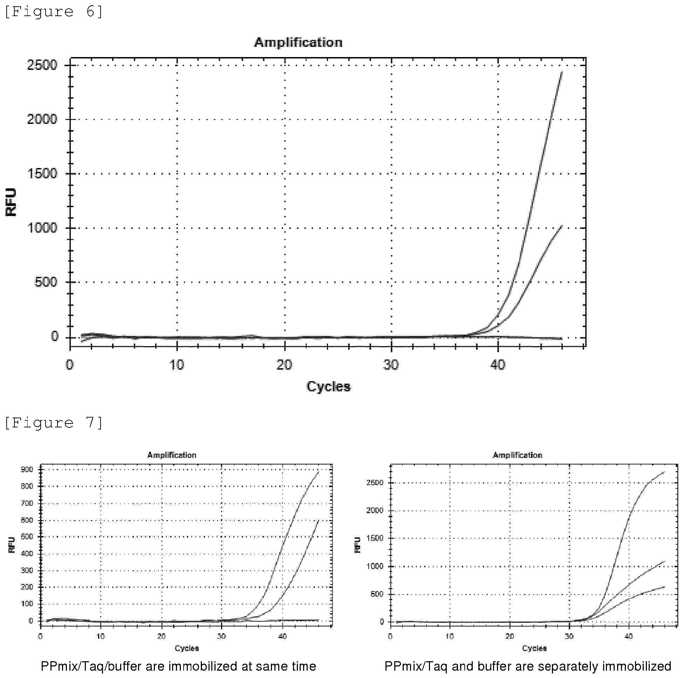

[Figure 6] Figure 6 is a diagram showing an amplification curve of real-time PCR assay in reaction container D (reaction container for a test sample contaminated with one copy of a positive control nucleic acid) in Example 5 of the present application. The abscissa depicts the number of reaction cycles, and the ordinate depicts fluorescence intensity RFU.
[Figure 7] Figure 7 is a diagram showing an amplification curve of real-time PCR assay in reaction container A (PPmix, etc., an enzyme, and a buffer are immobilized in one container) and reaction container B (PPmix, etc. and an enzyme are immobilized in one container, and a buffer (containing Mg²⁺ and dNTP) is immobilized in another container) in Example 6 of the present application. The abscissa depicts the number of reaction cycles, and the ordinate depicts fluorescence intensity RFU.

### Mode of Carrying Out the Invention

### 1. [Set comprising positive control nucleic acid and probe specific for the positive control nucleic acid]

The set comprising a positive control nucleic acid and a probe specific for the positive control nucleic acid (hereinafter, also simply referred to as a "probe for the positive control nucleic acid") (hereinafter, this set is simply referred to as the "set of the present invention") is intended for use in the detection or quantification of a target nucleic acid in a test sample using a primer set specific for the target nucleic acid (hereinafter, also simply referred to as a "primer set for the target nucleic acid") and a probe specific for the target nucleic acid (hereinafter, also simply referred to as a "probe for the target nucleic acid"). The positive control nucleic acid according to the present invention serves as a positive control of the target nucleic acid. The positive control nucleic acid of the present invention is useful in the detection of a target nucleic acid in a test sample. For example, by use of the positive control nucleic acid of the present invention, it can be confirmed that: the primer set for the target nucleic acid exerts intended functions so that nucleic acid amplification reaction is correctly performed; and the probe for the target nucleic acid exerts intended functions. The positive control nucleic acid of the present invention is also useful in the quantification (absolute quantification or relative quantification) of a target nucleic acid in a test sample. For use in the absolute quantification, for example, a calibration curve can be prepared on the basis of measurement results about known concentrations of a positive control nucleic acid to thereby accurately quantify an unknown concentration of a target nucleic acid in a test sample. For use in the relative quantification, for example, the number of cycles required to reach a given concentration is compared between a positive control nucleic acid sample and a test sample, and relative concentration difference can also be calculated on the basis of a PCR principle that a nucleic acid is amplified twice in one cycle.

### (Positive control nucleic acid according to present invention)

The positive control nucleic acid of the present invention is not particularly limited as long as the positive control nucleic acid is any nucleic acid selected from
(I) a single-stranded nucleic acid consisting of a nucleotide sequence having the following features (A) to (D),
(II) a single-stranded nucleic acid consisting of a nucleotide sequence complementary to the nucleotide sequence of the single-stranded nucleic acid (I), and
(III) a double-stranded nucleic acid formed from the single-stranded nucleic acid (I) and the single-stranded nucleic acid (II):
   (A) comprising nucleotide sequence A1 of a 5' primer specific for the target nucleic acid and nucleotide sequence A2 complementary to a 3' primer specific for the target nucleic acid;
   (B) comprising nucleotide sequence B1 complementary to the probe specific for the target nucleic acid, or nucleotide sequence B2 complementary to the nucleotide sequence B1;
   (C) comprising nucleotide sequence C1 complementary to the probe specific for the positive control nucleic acid, or nucleotide sequence C2 complementary to the nucleotide sequence C1; and
   (D) the nucleotide sequence B1 or B2 and the nucleotide sequence C1 or C2 are positioned between the nucleotide sequences A1 and A2.

The positive control nucleic acid of the present invention is, as mentioned above, a single-stranded nucleic acid or a double-stranded nucleic acid. Examples of such a nucleic acid include polynucleotides such as DNA and RNA. DNA is preferred from the viewpoint of stability.

The nucleotide sequence A1 in the feature (A) is not particularly limited as long as the nucleotide sequence A1 is the nucleotide sequence of a 5' primer specific for the target nucleic acid (hereinafter, also simply referred to as a "5' primer for the target nucleic acid"). The nucleotide sequence A2 in the feature (A) is not particularly limited as long as the nucleotide sequence A2 is a nucleotide sequence complementary to a 3' primer specific for the target nucleic acid (hereinafter, also simply referred to as a "3' primer for the target nucleic acid"). Provided that the positive control nucleic acid comprises the nucleotide sequences A1 and A2, the 5' primer for the target nucleic acid and the 3' primer for the target nucleic acid contained in the primer set for the target nucleic acid hybridize to the positive control nucleic acid so that the positive control nucleic acid can be amplified in nucleic acid amplification reaction to exert functions as a positive control. When the primer set for the target nucleic acid comprises two or more types of 5' primers for the target nucleic acid, the positive control nucleic acid comprises nucleotide sequences A1 as to all of these types of 5' primers for the target nucleic acid. When the primer set for the target nucleic acid comprises two or more types of 3' primers for the target nucleic acid, the positive control nucleic acid comprises nucleotide sequences A2 as to all of these types thereof.

The number of nucleotides of the nucleotide sequence A1 or A2 in the feature (A) is usually in the range of 15 to 50, preferably in the range of 17 to 35. These nucleotide sequences A1 and A2 may have the same number of nucleotides or may have different number of nucleotides. The number of nucleotides of the nucleotide sequence A1 or A2 is not necessarily required to be the same as the number of nucleotides of the corresponding nucleotide sequence of the primer for the target nucleic acid, but is preferably the same thereas.

The nucleotide sequence B1 in the feature (B) is not particularly limited as long as the nucleotide sequence B1 is a nucleotide sequence complementary to the probe for the target nucleic acid. The nucleotide sequence B2 in the feature (B) is not particularly limited as long as the nucleotide sequence B2 is a nucleotide sequence complementary to the nucleotide sequence B1. Provided that the positive control nucleic acid comprises the nucleotide sequence B1 or B2, the probe for the target nucleic acid can hybridize to an amplification product resulting from nucleic acid amplification reaction using the positive control nucleic acid as a template. Thus, for example, in the case of performing nucleic acid amplification reaction using the positive control nucleic acid in a reaction container for the positive control nucleic acid, by the detection or quantification of signals derived from the probe for the target nucleic acid, it can be confirmed that: the primer set for the target nucleic acid exerts intended functions so that the nucleic acid amplification reaction is correctly performed; and the probe for the target nucleic acid exerts intended functions, or a calibration curve indicating the relationship between the concentration of the positive control nucleic acid and signal intensity or the like can be prepared.

The number of nucleotides of the nucleotide sequence B1 or B2 in the feature (B) is preferably in the range of 15 to 100, more preferably in the range of 17 to 60, further preferably in the range of 20 to 40. These nucleotide sequences B1 and B2 may have the same number of nucleotides or may have different numbers of nucleotides. The number of nucleotides of the nucleotide sequence B1 or B2 is not necessarily the same as the number of nucleotides of the corresponding nucleotide sequence of the probe for the target nucleic acid, but is preferably the same.

The nucleotide sequence C1 in the feature (C) is not particularly limited as long as the nucleotide sequence C1 is a nucleotide sequence complementary to the probe specific for the positive control nucleic acid (probe for the positive control nucleic acid). The nucleotide sequence C2 in the feature (C) is not particularly limited as long as the nucleotide sequence C2 is a nucleotide sequence complementary to the nucleotide sequence C1. Provided that the positive control nucleic acid comprises the nucleotide sequence C1 or C2, the probe for the positive control nucleic acid can specifically hybridize to an amplification product resulting from nucleic acid amplification reaction using the positive control nucleic acid as a template. Thus, for example, in the case of performing nucleic acid amplification reaction in a reaction container for the test sample, the presence or absence of contamination of the reaction container for the test sample with the positive control nucleic acid can be confirmed by the detection of the presence or absence of signals derived from the probe for the positive control nucleic acid.

The number of nucleotides of the nucleotide sequence C1 or C2 in the feature (C) is preferably in the range of 15 to 100, more preferably in the range of 17 to 60, further preferably in the range of 20 to 40. These nucleotide sequences C1 and C2 may have the same number of nucleotides or may have different numbers of nucleotides. The number of nucleotides of the nucleotide sequence C1 or C2 is not necessarily the same as the number of nucleotides of the corresponding nucleotide sequence of the probe for the positive control nucleic acid, but is preferably the same.

In the case of using two or more types of positive control nucleic acids in combination, the nucleotide sequence C1 or C2 may differ among the types of positive control nucleic acids. From the viewpoint of detecting more types of positive control nucleic acids with fewer types of probes for the positive control nucleic acids, it is preferred that nucleotide sequences C1 or C2 having the same sequences should be used for two or more types of or all of the types of positive control nucleic acids among the positive control nucleic acids used in combination, and it is more preferred that the same nucleotide sequences C1 or C2 described above should be used for all of the types of positive control nucleic acids used in combination.

As described in the feature (D), in the positive control nucleic acid according to the present invention, the nucleotide sequence B1 or B2 and the nucleotide sequence C1 or C2 is positioned between the nucleotide sequences A1 and A2. By such positioning, an amplification product of the positive control nucleic acid comprises the nucleotide sequence B1 or B2 and the nucleotide sequence C1 or C2 so that the amplification product can be detected or quantified with the probe for the target nucleic acid or the probe for the positive control nucleic acid. When the primer set for the target nucleic acid comprises two or more types of 5' primers for the target nucleic acid and/or comprises two or more types of 3' primers for the target nucleic acid, the nucleotide sequence A1 in the feature (D) means nucleotide sequence A1 nearest the 5' end of the positive control nucleic acid, among two or more types of nucleotide sequences A1, and the nucleotide sequence A2 in the feature (D) means nucleotide sequence A2 nearest the 3' end of the positive control nucleic acid, among two or more types of nucleotide sequences A2.

In a preferred embodiment, examples of the positive control nucleic acid of the present invention can include a positive control nucleic acid having a nucleotide sequence altered from the nucleotide sequence of the target nucleic acid and, specifically, can preferably include a positive control nucleic acid in which the nucleotide sequence (I) is
(I') a nucleotide sequence wherein the nucleotide sequence C1 or C2 is inserted to a portion other than the nucleotide sequences A1 and A2 and the nucleotide sequence B1 or B2 in a single-stranded nucleic acid having the nucleotide sequences A1 and A2 and the nucleotide sequence B1 or B2 in a nucleotide sequence derived from the nucleotide sequence of the target nucleic acid, or
a nucleotide sequence wherein one or more (preferably 1 to 200, more preferably 1 to 100, further preferably 1 to 50) nucleotide sequence(s) of a portion other than the nucleotide sequences A1 and A2 and the nucleotide sequence B1 or B2 is substituted with the nucleotide sequence C1 or C2 in a single-stranded nucleic acid having the nucleotide sequences A1 and A2 and the nucleotide sequence B1 or B2 in a nucleotide sequence derived from the nucleotide sequence of the target nucleic acid.

Among others, examples thereof can more preferably include a positive control nucleic acid in which the nucleotide sequence of the single-stranded nucleic acid (I) is
(I'') a nucleotide sequence wherein the nucleotide sequence C1 or C2 is inserted to a portion other than the nucleotide sequences A1 and A2 and the nucleotide sequence B1 or B2 in a single-stranded nucleic acid having the nucleotide sequences A1 and A2 and the nucleotide sequence B1 or B2 in a nucleotide sequence derived from the nucleotide sequence of the target nucleic acid.

The positive control nucleic acid according to the present invention can be prepared by synthesis, nucleic acid amplification reaction, cloning, or a combination thereof according to a routine method and is preferably prepared by synthesis from the viewpoint of convenience. Such preparation of the positive control nucleic acid can also be commissioned to a company manufacturing artificial genes or the like.

### (Probe specific for positive control nucleic acid according to present invention)

The probe specific for the positive control nucleic acid (probe for the positive control nucleic acid) of the present invention means a probe that specifically hybridizes to a portion of the positive control nucleic acid and permits specific detection or quantification of the positive control nucleic acid of the present invention. The nucleotide sequence of this probe for the positive control nucleic acid is not particularly limited as long as the nucleotide sequence is a nucleotide sequence that has 70% or lower, preferably 68% or lower, more preferably 65% or lower, further preferably 62% or lower sequence identity to the sequence of any portion of the genomes of an organism species from which the test sample is derived and an organism species from which the target nucleic acid is derived, and transcripts thereof, and has a Tm value of 65°C or lower, preferably 60°C or lower, more preferably 55°C or lower, even more preferably 50°C or lower, further preferably 47°C or lower, still further preferably 45°C or lower, still further preferably 43°C or lower. Provided that the probe for the positive control nucleic acid has such a nucleotide sequence, the probe for the positive control nucleic acid can be avoided from hybridizing to a nucleic acid other than the positive control nucleic acid. As a result, the probe for the positive control nucleic acid specifically hybridizes to the positive control nucleic acid.

The probe for the positive control nucleic acid of the present invention is usually preferably a single-stranded nucleic acid. Examples of such a nucleic acid include polynucleotides such as DNA and RNA. DNA is preferred from the viewpoint of stability.

It is preferred that the probe for the positive control nucleic acid should have 70% or lower, preferably 68% or lower, more preferably 65% or lower, further preferably 62% or lower sequence identity to the sequence of any portion of the genomes of not only the organism species from which the test sample is derived and the organism species from which the target nucleic acid is derived but more organism species, and transcripts thereof. This is because use of such a sequence as the sequence of the probe for the positive control nucleic acid offers a more versatile positive control nucleic acid that can be used even when the organism species from which the test sample is derived is of different type therefrom or the target nucleic acid is of different type therefrom. Examples of the "more organism species" mentioned above can include 2 or more species, preferably 4 or more species, more preferably 10 or more species, even more preferably 20 or more species, further preferably 50 or more species, still further preferably 100 or more species, still further preferably 500 or more species, still further preferably 1000 or more species, still further preferably 5000 or more species, still further preferably 7500 or more species, still further preferably 10000 or more species, still further preferably 12500 or more species, still further preferably 15000 or more species, selected from the group consisting of mammals, bacteria, fungi, and viruses, preferably the group consisting of mammals, birds, reptiles, amphibians, fishes, bacteria, fungi, and viruses. The sequences of the genomes of these organism species or transcripts thereof can be confirmed by examining, for example, sequence information of a sequence database such as GenBank.

In the present invention, the organism species from which the test sample is derived and the organism species from which the target nucleic acid is derived may be different species or may be the same species. Examples of the case of being different species include the case where, for the purpose of confirming whether a mammal is infected by a pathogenic microbe, a particular nucleic acid of the pathogenic microbe is used as a target nucleic acid, and a test sample derived from the mammal is used. Examples of the case of being the same species include the case where, for the purpose of confirming whether a mammal is affected by a disease, a particular nucleic acid of a mammalian gene related to the disease is used as a target nucleic acid, and a test sample derived from the mammal is used.

The probe for the positive control nucleic acid is a nucleotide sequence that has a Tm value of 65°C or lower, preferably 60°C or lower, more preferably 55°C or lower, even more preferably 50°C or lower, further preferably 47°C or lower, still further preferably 45°C or lower, still further preferably 43°C or lower. The Tm value of a certain nucleotide sequence can be calculated by a calculation method known in the art such as nearest neighbor method, Wallace method, or GC% method. Among them, calculation by the nearest neighbor method can be preferred. In the present invention, the Tm value (melting temperature) of a certain nucleotide sequence means a temperature at which, when the temperature of a solution containing double-stranded nucleic acids each consisting of the nucleotide sequence and a nucleotide sequence complementary thereto is elevated, half the total number of these double-stranded nucleic acids mentioned above dissociates.

Examples of a preferred method for selecting the nucleotide sequence of the probe for the positive control nucleic acid of the present invention can preferably include a selection method comprising the following steps:
step (i) : the step of obtaining the group consisting of repeat nucleotide sequences having 4 to 16(preferably 4 to 12, more preferably 4 to 8, further preferably 4 to 6) nucleotides obtained by repeating a nucleotide sequence having 2 to 4 (preferably 2 to 3, more preferably 2) nucleotides twice to four times (preferably twice or three times, more preferably twice);
step (ii): the step of linking two or more repeat nucleotide sequences selected from the group obtained in the step (i) to establish a candidate nucleotide sequence;
step (iii): the step of confirming, by use of, for example, sequence information of a sequence database, whether the candidate nucleotide sequence established in the step (ii) or a candidate nucleotide sequence confirmed to satisfy a condition in step (iv) mentioned later satisfies the condition of being a nucleotide sequence that has 70% or lower, preferably 68% or lower, more preferably 65% or lower, further preferably 62% or lower sequence identity to the sequence of any portion of the genomes of an organism species from which the test sample is derived and an organism species from which the target nucleic acid is derived, and transcripts thereof;
step (iv) : the step of confirming whether the candidate nucleotide sequence established in the step (ii) or the candidate nucleotide sequence confirmed to satisfy the condition in the step (iii) satisfies the condition of being a nucleotide sequence that has a Tm value of 65°C or lower, preferably 60°C or lower, more preferably 55°C or lower, even more preferably 50°C or lower, further preferably 47°C or lower, still further preferably 45°C or lower, still further preferably 43°C or lower; and
step (v) : the step of selecting the candidate nucleotide sequence that satisfies both of the conditions of the step (iii) and the step (iv) as the nucleotide sequence of the probe for the positive control nucleic acid.

Examples of the "nucleotide sequence having 2 nucleotides" in the step (i) include AT, AG, AC, TA, TG, TC, GA, GT, GC, CA, CT, and CG. Examples of the "nucleotide sequence having 3 nucleotides" include ATA, ATG, ATC, AGA, AGT, AGC, ACA, ACT, ACG, TAT, TAG, TAC, TGA, TGT, TGC, TCA, TCT, TCG, GAT, GAG, GAC, GTA, GTG, GTC, GCA, GCT, GCG, CAT, CAG, CAC, CTA, CTG, CTC, CGA, CGT, and CGC.

For the repeat nucleotide sequence having 4 to 16 nucleotides obtained by repeating a nucleotide sequence having 2 to 4 nucleotides twice to four times, only one nucleotide sequence having 2 to 4 nucleotides may be used in the repeating, or two or more nucleotide sequences having 2 to 4 nucleotides may be used in the repeating, as long as the repeat nucleotide sequence having 4 to 16 nucleotides is a nucleotide sequence obtained by repeating a nucleotide sequence having 2 to 4 nucleotides twice to four times. The nucleotide sequences for use in the repeating may have different numbers of nucleotides in the range of 2 to 4. Preferably, nucleotide sequences having the same number of nucleotides are used in the repeating. The "group consisting of repeat nucleotide sequences having 4 to 16 nucleotides obtained by repeating a nucleotide sequence having 2 to 4 nucleotides twice to four times" includes every type of repeat nucleotide sequence having 4 to 16 nucleotides theoretically obtained by such repeating.

Preferred examples of the candidate nucleotide sequence in the step (i) include a "repeat nucleotide sequence having 4 nucleotides obtained by repeating a nucleotide sequence having 2 nucleotides twice", a "repeat nucleotide sequence having 6 nucleotides obtained by repeating a nucleotide sequence having 2 nucleotides three times", and a "repeat nucleotide sequence having 6 nucleotides obtained by repeating a nucleotide sequence having 3 nucleotides twice". Among others, more preferred examples thereof include a "repeat nucleotide sequence having 4 nucleotides obtained by repeating a nucleotide sequence having 2 nucleotides twice" and a "repeat nucleotide sequence having 6 nucleotides obtained by repeating a nucleotide sequence having 2 nucleotides three times".

Examples of the "repeat nucleotide sequence having 4 nucleotides obtained by repeating a nucleotide sequence having 2 nucleotides twice" include ATAT, AGAG, ACAC, TATA, TGTG, TCTC, GAGA, GTGT, GCGC, CACA, CTCT, and CGCG. Examples of the "repeat nucleotide sequence having 6 nucleotides obtained by repeating a nucleotide sequence having 2 nucleotides three times" include ATATAT, AGAGAG, ACACAC, TATATA, TGTGTG, TCTCTC, GAGAGA, GTGTGT, GCGCGC, CACACA, CTCTCT, and CGCGCG. Examples of the "repeat nucleotide sequence having 6 nucleotides obtained by repeating a nucleotide sequence having 3 nucleotides twice" include ATAATA, ATGATG, ATCATC, AGAAGA, AGTAGT, AGCAGC, ACAACA, ACTACT, ACGACG, TATTAT, TAGTAG, TACTAC, TGATGA, TGTTGT, TGCTGC, TCATCA, TCTTCT, TCGTCG, GATGAT, GAGGAG, GACGAC, GTAGTA, GTGGTG, GTCGTC, GCAGCA, GCTGCT, GCGGCG, CATCAT, CAGCAG, CACCAC, CTACTA, CTGCTG, CTCCTC, CGACGA, CGTCGT, and CGCCGC.

The step (ii) is not particularly limited as long as the step is of linking two or more repeat nucleotide sequences selected from the group obtained in the step (i) to establish a candidate nucleotide sequence. The order in which the two or more repeat nucleotide sequences are linked may be random. Preferably, the two or more repeat nucleotide sequences are linked such that repeat nucleotide sequences adjacent to each other are of different types. The number of candidate nucleotide sequences to be linked can be appropriately set in consideration of the number of nucleotides of the candidate nucleotide sequence, and the number of nucleotides of the nucleotide sequence of the probe for the positive control nucleic acid of the present invention.

Specific examples of a method for confirming whether the candidate nucleotide sequence satisfies the condition in the step (iii) include a method of confirming whether the candidate nucleotide sequence established in the step (ii) or a candidate nucleotide sequence confirmed to satisfy a condition in step (iv) mentioned later satisfies the condition in the step (iii), by use of sequence information of a sequence database (GenBank, etc.), software for homology search (e.g., BLAST(R)), and the like. In a preferred embodiment, examples of such a method include a method of performing BLAST homology search under condition A given below. By such homology search, sequence identity to all sequences to be searched can be determined, and whether the highest sequence identity is, for example, 70% or lower can be confirmed.

### (Condition A)

Databases 1) to 3) are selected in this order to perform homology search.
Program: Nucleotide Blast
Database: 1) Human genomic + transcript; 2) Mouse genomic + transcript; and 3) Others (nr etc.);
Optimize for Highly similar sequence (Megablast)

By the BLAST homology search under the condition A, sequence identity to the sequences of the genomes of up to approximately 160000 organism species, and transcripts thereof can be confirmed.

Specific examples of a method for confirming whether the candidate nucleotide sequence satisfies the condition in the step (iv) include a method of confirming whether the candidate nucleotide sequence established in the step (ii) or the candidate nucleotide sequence confirmed to satisfy the condition in the step (iii) satisfies the condition in the step (iv), through calculation by a calculation method known in the art such as nearest neighbor method, Wallace method, or GC% method (preferably nearest neighbor method). Such calculation of Tm can be conveniently conducted using, for example, software on a website.

Preferred examples of the nucleotide sequence of the probe for the positive control nucleic acid of the present invention selected by actually performing the selection method comprising the steps (i) to (v) including the BLAST homology search under the condition A include the nucleotide sequences of SEQ ID NOs: 1 to 3 and the nucleotide sequence of SEQ ID NO: 15. The sequence identity (%) and Tm values (°C) of the nucleotide sequences of SEQ ID NOs: 1 to 3 are as shown in Table 1 mentioned later.

The number of nucleotides of the probe for the positive control nucleic acid is not particularly limited as long as the probe for the positive control nucleic acid is capable of specifically hybridizing to the positive control nucleic acid. Depending on the nucleic acid amplification method used, the number of nucleotides of the probe for the positive control nucleic acid is preferably in the range of 13 to 100, more preferably in the range of 17 to 60, further preferably in the range of 20 to 30.

In the case of using two or more types of positive control nucleic acids, the probe for the positive control nucleic acid used may differ among the positive control nucleic acids. From the viewpoint of detecting more types of positive control nucleic acids with fewer types of probes for the positive control nucleic acids, it is preferred that the same nucleotide sequences should be used in two or more types of or all of the types of probes for the positive control nucleic acids among the probes for the positive control nucleic acids used in combination, and it is more preferred that the same nucleotide sequences should be used in all of the types of probes for the positive control nucleic acids used in combination.

It is preferred that the probe for the positive control nucleic acid of the present invention should be labeled with a labeling material for the detection or quantification of the corresponding positive control nucleic acid. It is more preferred that the probe for the positive control nucleic acid should be labeled with a fluorescent material, from the viewpoint of more rapid or more highly sensitive detection or quantification. Examples of the labeling material other than the fluorescent material include biotin, a complex of biotin and avidin, and enzymes such as peroxidase. Preferred examples of the fluorescent material include fluorescent proteins such as luciferase as well as fluorescent dyes such as FITC (fluorescein isothiocyanate), 6-FAM (6-carboxyfluorescein), TET (6-carboxy-4,7,2',7'-tetrachlorofluorescein), JOE (6-carboxy-4',5'-dichloro-2',7'-dimethoxyfluorescein), Cy3, Cy5, and HEX (4,7,2',4',5,',7'-hexachloro-6-carboxyfluorescein). It is more preferred that the probe for the target nucleic acid according to the present invention should be double-labeled with a fluorescent material (reporter fluorescent dye) and a quenching material (quencher fluorescent dye). Examples of the reporter fluorescent dye include the fluorescent dye mentioned above. Examples of the quencher fluorescent dye include: rhodamine fluorescent dyes such as 6-carboxytetramethylrhodamine (TAMRA) and 6-carboxy-X-rhodamine (ROX); and blackhole quenchers such as BHQ-1 ([(4-(2-nitro-4-methyl-phenyl)-azo)-yl-((2-methoxy-5-methyl-phenyl)-azo)]-anline) and BHQ-2([(4-(1-nitrophenyl)-azo)-yl-((2,5-dimethoxy-phenyl)-azo)]-anline). Because the probe for the positive control nucleic acid and the probe for the target nucleic acid can be detected or quantified conveniently and rapidly at the same time, it is preferred that a label (preferably a fluorescent label) different from the label (preferably a fluorescent label) for use in the probe for the target nucleic acid should be used as the label (preferably a fluorescent label) for the probe for the positive control nucleic acid.

Examples of the type of the probe for the positive control nucleic acid according to the present invention include, but are not particularly limited to, TaqMan(R) probes, molecular beacon probes, cycling probes, Eprobe (R), Qprobe (R), scorpion probes, and hybridization-probes. Among others, a TaqMan probe is preferred. The TaqMan probe is usually a linear oligonucleotide of a nucleic acid probe modified at its 5' end with a fluorescent material (reporter fluorescent dye) and modified at its 3' end with a quenching material (quencher fluorescent dye). The molecular beacon probe is usually an oligonucleotide of a nucleic acid probe modified at its 5' end with a fluorescent material (reporter fluorescent dye) and modified at its 3' end with a quenching material (quencher fluorescent dye), and is capable of assuming a stem-loop structure. The cycling probe is usually a chimeric oligonucleotide consisting of RNA and DNA, and is an oligonucleotide modified at its one end with a fluorescent material (reporter fluorescent dye) and modified at the other end with a quenching material (quencher fluorescent dye). The Eprobe is usually an artificial nucleic acid with a thymine base having two fluorescent dyes, and emits fluorescence when bound with a target, while the emission of its fluorescence is suppressed in a single-stranded state that is not bound with the target. The Qprobe is usually an oligonucleotide having terminal cytosine, and the terminal cytosine is labeled with a fluorescent material. The Qprobe is also called guanine-quenched probe. The scorpion probe is usually an oligonucleotide of a nucleic acid probe modified at its one end with a fluorescent material (reporter fluorescent dye) and modified at its 3' end with a quenching material (quencher fluorescent dye), and is capable of assuming a hairpin-loop structure. The hybridization-probe is constituted by a donor probe consisting of an oligonucleotide modified at its 3' end with a fluorescent dye, and an acceptor probe consisting of an oligonucleotide modified at its 5' end with a fluorescent dye. Upon binding of these probes to a target, both of the fluorescent dyes are located in proximity to each other so that the fluorescent dye of the acceptor probe is excited by fluorescence from the fluorescent dye of the donor probe to emit light.

The probe for the positive control nucleic acid according to the present invention can be prepared by synthesis, nucleic acid amplification reaction, cloning, or a combination thereof according to a routine method and is preferably prepared by synthesis from the viewpoint of convenience. Such preparation of the probe for the target nucleic acid can also be commissioned to a company manufacturing oligonucleotide probes.

### (Set comprising positive control nucleic acid and probe for the positive control nucleic acid)

The set of the present invention comprises a positive control nucleic acid and a probe for the positive control nucleic acid. The set of the present invention comprises one or more type(s), preferably two or more types, more preferably two or more types and four or less types of positive control nucleic acids. The set of the present invention comprising two or more types of positive control nucleic acids is preferably used in one reaction container or used in each of two or more different reaction containers because two or more types of target nucleic acids in a test sample can be detected or quantified. Particularly, these two or more types of positive control nucleic acids are more preferably used in combination in one reaction container because two or more types of target nucleic acids in a test sample can be detected or quantified at the same time in fewer reaction containers. When the set of the present invention comprises two or more types of positive control nucleic acids, it is preferred that at least one type of positive control nucleic acid should be a positive control nucleic acid for a nucleic acid of a housekeeping gene as a target nucleic acid. Use of at least one type of positive control nucleic acid for a housekeeping gene as a target nucleic acid is useful in the measurement of the amounts of target nucleic acids with respect to a particular amount of the test sample or the correction of variations in the amount of the test sample among reaction containers, because the amount of a test sample (e.g., the number of test cells or the amount of genomic nucleic acids) in the test sample can be predicted.

The set of the present invention comprise one or more type (s) of probe for the positive control nucleic acid. The number of the type of the probe for the positive control nucleic acid contained in the set of the present invention may be the same as the number of the type of the positive control nucleic acid. From the viewpoint that more types of positive control nucleic acids are detected or quantified with fewer types of probes for the positive control nucleic acids, it is preferred to use at least one type of probe for the positive control nucleic acids that permits detection or quantification of two or more types of or all of the types of positive control nucleic acids among the positive control nucleic acids used in combination, and it is more preferred to use one type of probe for the positive control nucleic acids that permits detection or quantification of all of the types of positive control nucleic acids used in combination.

### (Target nucleic acid according to present invention)

The target nucleic acid according to the present invention means a nucleic acid to be detected or quantified from a test sample, and more specifically means a nucleic acid of a region that is amplified by nucleic acid amplification reaction using a "primer set specific for the target nucleic acid" mentioned later. The target nucleic acid can be appropriately selected according to a purpose such as the diagnosis of a disease or the determination of the risk of developing a disease. Examples thereof include a gene, etc. (also including a noncoding region) related to the cause or exacerbation of a disease or a portion thereof, or their transcripts, a gene, etc. (also including a noncoding region) abnormally expressed by the development of a disease or a portion thereof, or their transcripts, and a gene, etc. (also including a noncoding region) related to the risk of developing a disease or a portion thereof, or their transcripts. For the target nucleic acid, it is also preferred to select a portion that permits specific detection or quantification of the target nucleic acid in the detection or quantification from a test sample. Such selection can be carried out by examining the sequences of the genomes of the organism species from which the test sample is derived and the organism species from which the target nucleic acid is derived, and transcripts thereof, the nucleotide sequences of candidate genes, and the like by use of, for example, sequence information of a sequence database, selecting a candidate sequence of the target nucleic acid (target nucleic acid candidate sequence) from among these nucleotide sequences, and conducting homology search by BLAST or the like as to the target nucleic acid candidate sequence. When the target nucleic acid candidate sequence is, for example, a sequence selected from the genome of the organism species (e.g., a human) from which the test sample is derived, or a transcript thereof, a target nucleic acid candidate sequence having low sequence identity to the "genome or a transcript thereof", except for the portion of the target nucleic acid candidate sequence, of at least the organism species can be selected as the target nucleic acid. On the other hand, when the target nucleic acid candidate sequence is a sequence selected from the genome of an organism species (e.g., a virus) other than the organism species (e.g., a human) from which the test sample is derived, or a transcript thereof, a candidate sequence having low sequence identity to the genome of at least the organism species (e.g., a human) from which the test sample is derived or a transcript thereof, and low sequence identity to the "genome or a transcript thereof", except for the portion of the target nucleic acid candidate sequence, of the organism species (e.g., a virus) from which the target nucleic acid is derived can be selected as the target nucleic acid.

Provided that the target nucleic acid is a portion that permits specific detection or quantification of the gene, etc. related to the cause or exacerbation of a disease, the gene, etc. abnormally expressed by the development of a disease, and the gene, etc. related to the risk of developing a disease as mentioned above, the presence or absence of the target nucleic acid in a test sample can be detected, or the target nucleic acid can be quantified to thereby confirm or diagnose whether or not a test organism from which the test sample is derived is affected by the disease, or the severity thereof, or to thereby determine the degree of the risk of developing the disease in the test organism. Specific examples of these genes, etc. preferably include genes, etc. of the following viruses or bacteria that may be causative of the disease, for example.

Examples of the viruses include: RNA viruses such as human immunodeficiency virus type 1 (HIV1) and type 2 (HIV2), human adult T-cell leukemia/lymphoma virus type 1 (HTLV1) and type 2 (HTLV2), hepatitis C virus (HCV), picornaviruses, caliciviruses, orthomyxoviruses, and togaviruses; and DNA viruses such as BK virus (BKV), JC virus (JCV), cytomegalovirus (CMV), EB virus (EBV), human herpesvirus type 6 (HHV6), herpes simplex virus (HSV), varicella zoster (VZV), poxviruses, parvoviruses, papovaviruses, hepatitis B virus (HBV), adenoviruses, and human papilloma virus (HPV).

Examples of the bacteria include a bacterium of the genus *Salmonella* (*Salmonella typhi, Salmonella paratyphi* A, *Salmonella paratyphi* B, *Salmonella enteritidis, Salmonella typhimurium, Salmonella arizonae,* etc.), a bacterium of the genus *Shigella* (*Shigella* spp., etc.), a bacterium of the genus *Vibrio* (*Vibrio parahaemolyticus, Vibrio cholerae,* non-agglutinable (NAG) vibrio, *Vibrio mimicus, Vibrio vulnificus, Vibrio alginolyticus,* etc.), a bacterium of the genus *Aeromonas* (*Aeromonas hydrophila, Aeromonas sobria, Aeromonas caviae, Aeromonas salmonicida,* etc.), a bacterium of the genus *Plesiomonas* (*Plesiomonas shigelloides,* etc.), a bacterium of the genus *Campylobacter (Campylobacter jejuni, Campylobacter coli, Campylobacter fetus,* etc.), a bacterium of the genus *Clostridium (Clostridium perfringens, Clostridium botulinum,* etc.), a bacterium of the genus *Staphylococcus* (*Staphylococcus aureus,* MRSA, etc.), a bacterium of the genus *Escherichia* (*Escherichia coli,* enterohemorrhagic *Escherichia coli* (0157, etc.), enterotoxigenic *Escherichia coli,* enteroinvasive *Escherichia coli,* enteropathogenic *Escherichia coli,* enteroadherent *Escherichia coli,* etc.), a bacterium of the genus *Yersinia* (*Yersinia enterocolitica, Yersinia pseudotuberculosis, Yersinia pestis,* etc.), a bacterium of the genus *Bacillus* (*Bacillus cereus, Bacillus anthracis, Bacillus subtilis, Bacillus stearothermophilus,* etc.), a bacterium of the genus *Listeria* (*Listeria monocytogenes,* etc.), a bacterium of the genus *Mycobacterium (Mycobacterium tuberculosis, Mycobacterium bovis, Mycobacterium avium,* etc.), a bacterium of the genus *Treponema* (*Treponema pallidum*), and a bacterium of the genus *Neisseria* (*Neisseria gonorrhoeae*). Examples of fungi that may be causative of the disease include a fungus of the genus *Candida,* a fungus of the genus *Aspergillus,* a fungus of the genus *Mucor,* a fungus of the genus *Cryptococcus,* and a fungus of the genus *Pneumocystis.*

Specific examples of the target nucleic acid according to the present invention can include a polynucleotide consisting of a partial nucleotide sequence (preferably a nucleotide sequence of nucleotide positions 1889 to 2028 of SEQ ID NO: 4: SEQ ID NO: 5) of BALF5 gene (nucleotide sequence of nucleotide positions 153699 to 156746 of GenBank Accession No. V01555: SEQ ID NO: 4) that permits specific detection or quantification of EBV, a polynucleotide consisting of a partial nucleotide sequence of hemagglutinin gene or neuraminidase gene that permits specific detection or quantification of influenza virus, a polynucleotide consisting of a partial nucleotide sequence of invA gene that permits specific detection or quantification of a bacterium of the genus *Salmonella,* a polynucleotide consisting of a partial nucleotide sequence of UL83 gene that permits specific detection or quantification of cytomegalovirus (CMV), a polynucleotide consisting of a partial nucleotide sequence of hexon protein gene that permits specific detection or quantification of adenovirus (ADV), a polynucleotide consisting of a partial nucleotide sequence of UL27 gene that permits specific detection or quantification of herpes simplex virus type 1 (HSV1), a polynucleotide consisting of a partial nucleotide sequence of U57 gene that permits specific detection or quantification of human herpes virus type 6 (HHV6), a polynucleotide consisting of a partial nucleotide sequence of NS1 gene or NS2 gene that permits specific detection or quantification of parvovirus B19 (pB19), a polynucleotide consisting of a partial nucleotide sequence of rrsA to rrLA genes that permits specific detection or quantification of mycoplasma, a polynucleotide consisting of a partial nucleotide sequence of gag gene, pol gene, or LTR gene that permits specific detection or quantification of human immunodeficiency virus (HIV), a polynucleotide consisting of a partial nucleotide sequence of NS1 gene or NCR gene that permits specific detection or quantification of hepatitis C virus, and a polynucleotide consisting of a partial nucleotide sequence of S gene or X gene that permits specific detection or quantification of hepatitis B virus.

The target nucleic acid according to the present invention may be a housekeeping gene or a portion thereof, in addition to a gene related to the cause or exacerbation of a disease or a portion thereof, a gene abnormally expressed by the development of a disease or a portion thereof and a gene related to the risk of developing a disease or a portion thereof, etc. as mentioned above. Use of a housekeeping gene or a portion thereof as the target nucleic acid is useful in the measurement of the amounts of target nucleic acids with respect to a particular amount of the test sample or the correction of variations in the amount of the test sample among reaction containers, because the amount of a test sample (e.g., the number of test cells or the amount of genomic nucleic acids) in the test sample can be predicted.

Examples of the housekeeping gene can include TBP (TATA-box binding protein) gene, GAPDH (glyceraldehyde-3-phosphate dehydrogenase) gene, 18S rRNA gene, ACTB (β-actin) gene, ALAS (5-aminolevulinate synthase) gene, β2M (β2 microglobulin) gene, β-globin gene, G6PD (glucose-6-phosphate dehydrogenase) gene, GUSB (β-glucuronidase) gene, HPRT1 (hypoxanthine phosphoribosyltransferase 1) gene, IP08 (importin 8) gene, PBGD (porphobilinogen deaminase) gene, PGK1 (phosphoglycerate kinase 1) gene, PPIA (peptidylprolyl isomerase A) gene, RPL13A (ribosomal protein L13a) gene, RPLP0 (ribosomal protein large P0) gene, SDHA (succinate dehydrogenase subunit A) gene, TFRC (transferrin receptor) gene, and YWHAZ (3-monooxygenase/tryptophan 5-monooxygenase activation protein, zeta) gene. Among others, TBP gene can be preferred.

When the target nucleic acid is a housekeeping gene or a portion thereof, as with the case where the target nucleic acid is a gene related to the cause or exacerbation of a disease or a portion thereof, etc., it is preferred to select a portion that permits specific detection or quantification of the target nucleic acid in the detection or quantification from a test sample. The nucleotide sequence of the housekeeping gene is known in the art. Therefore, such selection can be carried out by examining the sequences of the genomes of the test organism species from which the test sample is derived and the organism species from which the target nucleic acid is derived, and transcripts thereof, the nucleotide sequences of candidate genes, and the like by use of, for example, sequence information of a sequence database, selecting a candidate sequence of the target nucleic acid (target nucleic acid candidate sequence) from among these nucleotide sequences, and conducting homology search by BLAST or the like as to the target nucleic acid candidate sequence. When the target nucleic acid candidate sequence is, for example, a sequence selected from the genome of the organism species (e.g., a human) from which the test sample is derived, or a transcript thereof, a target nucleic acid candidate sequence having low sequence identity to the "genome or a transcript thereof", except for the portion of the target nucleic acid candidate sequence, of at least the organism species can be selected as the target nucleic acid. On the other hand, when the target nucleic acid candidate sequence is a sequence selected from the genome of an organism species (e.g., a virus) other than the organism species (e.g., a human) from which the test sample is derived, or a transcript thereof, a candidate sequence having low sequence identity to the genome of at least the organism species (e.g., a human) from which the test sample is derived or a transcript thereof, and low sequence identity to the "genome or a transcript thereof", except for the portion of the target nucleic acid candidate sequence, of the organism species (e.g., a virus) from which the target nucleic acid is derived can be selected as the target nucleic acid.

Examples of the type of the target nucleic acid according to the present invention include nucleic acids such as DNA and RNA. Among others, preferred examples thereof include DNA and RNA. Examples of the DNA include genomic DNA, cDNA, and synthetic DNA. Examples of the RNA include total RNA, mRNA, rRNA, siRNA, hnRNA, piRNA, aRNA, miRNA, and synthetic RNA. The number of nucleotides of the target nucleic acid is not particularly limited and is preferably in the range of 60 to 500, more preferably in the range of 70 to 300. The target nucleic acid according to the present invention may be double-stranded or may be single-stranded.

### 2. [Kit comprising primer set specific for target nucleic acid, probe specific for target nucleic acid, positive control nucleic acid, probe specific for positive control nucleic acid, and two or more reaction containers]

The kit of the present invention is a kit for use in the detection or quantification of a target nucleic acid in a test sample. Use of the kit of the present invention can simplify the operation of reagents such as primers and probes in the detection or quantification of a target nucleic acid. Therefore, a target nucleic acid in a test sample can be detected or quantified more conveniently and rapidly while the presence or absence of contamination of a container for the test sample with a positive control nucleic acid is conveniently and rapidly confirmed. The kit of the present invention can also be preserved, for example, for 6 months or longer, at ordinary temperature. Therefore, the detection or quantification of a target nucleic acid can be immediately performed when needed as long as the kit of the present invention is kept handy.

The kit of the present invention is not particularly limited as long as the kit comprises: a primer set specific for the target nucleic acid (primer set for the target nucleic acid); a probe specific for the target nucleic acid (probe for the target nucleic acid); the set of the present invention comprising a positive control nucleic acid and a probe specific for the positive control nucleic acid; and two or more reaction containers, wherein
the primer set specific for the target nucleic acid, the probe specific for the target nucleic acid, and the probe specific for the positive control nucleic acid are immobilized in at least one reaction container (s) for the test sample among the two or more reaction containers, and the primer set specific for the target nucleic acid, the probe specific for the target nucleic acid, the positive control nucleic acid, and the probe specific for the positive control nucleic acid are immobilized in at least one of the remaining reaction container(s) for the positive control nucleic acid.

Reagents, except for the primer set and the positive control nucleic acid, necessary for the nucleic acid amplification reaction of the target nucleic acid may be immobilized in a reaction container for the test sample and a reaction container for the positive control nucleic acid, from the viewpoint of more conveniently and rapidly detecting or quantifying the target nucleic acid. Examples of the reagents except for the primer set and the positive control nucleic acid include one or more (preferably three or more, more preferably all of four) reagent(s) selected from the group consisting of a nucleic acid-polymerizing enzyme (e.g., polymerase), a substance serving as a material for nucleic acids (e.g., dNTP), a buffer for nucleic acid amplification reaction (e.g., a component having a buffering effect, such as Tris-Cl, and a surfactant such as Tween 20), and Mg²⁺. These reagents except for the primer set and the positive control nucleic acid can be commercially available. The nucleic acid-polymerizing enzyme, the buffer for nucleic acid amplification reaction, the substance serving as a material for nucleic acids, and Mg²⁺ may be immobilized in the same reaction container as that for the primer set or the positive control nucleic acid. From the viewpoint of obtaining a kit having higher preservation stability at room temperature, it is preferred that one or more reagent(s) selected from the group consisting of the nucleic acid-polymerizing enzyme, the buffer for nucleic acid amplification reaction (preferably the nucleic acid-polymerizing enzyme and the buffer for nucleic acid amplification reaction), the substance serving as a material for nucleic acids, and Mg²⁺, more preferably the nucleic acid-polymerizing enzyme and/or the buffer for nucleic acid amplification reaction (the buffer is preferably a buffer containing the substance serving as a material for nucleic acids and Mg²⁺), further preferably the nucleic acid-polymerizing enzyme or the buffer for nucleic acid amplification reaction (the buffer is preferably a buffer containing the substance serving as a material for nucleic acids and Mg²⁺), should be immobilized in a reaction container different from that for the primer set or the positive control nucleic acid, or should be immobilized in none of the reaction containers. From the viewpoint of more conveniently and rapidly detecting or quantifying the target nucleic acid, it is more preferred that one or more reagent(s) selected from the group consisting of the nucleic acid-polymerizing enzyme, the buffer for nucleic acid amplification reaction (preferably the nucleic acid-polymerizing enzyme and the buffer for nucleic acid amplification reaction), the substance serving as a material for nucleic acids, and Mg²⁺, more preferably the nucleic acid-polymerizing enzyme and/or the buffer for nucleic acid amplification reaction (the buffer is preferably a buffer containing the substance serving as a material for nucleic acids and Mg²⁺), further preferably the nucleic acid-polymerizing enzyme or the buffer for nucleic acid amplification reaction (the buffer is preferably a buffer containing the substance serving as a material for nucleic acids and Mg²⁺), should be immobilized in a reaction container different from that for the primer set or the positive control nucleic acid. When at least the nucleic acid-polymerizing enzyme and the buffer for nucleic acid amplification reaction (the buffer is preferably a buffer containing the substance serving as a material for nucleic acids and Mg²⁺) are immobilized in a reaction container different from that for the primer set or the positive control nucleic acid, it is further preferred that the nucleic acid-polymerizing enzyme and the buffer for nucleic acid amplification reaction (the buffer is preferably a buffer containing the substance serving as a material for nucleic acids and Mg²⁺) should be immobilized in separate reaction containers.

The two or more reaction containers in the kit of the present invention are not particularly limited by material, size, shape, etc. as long as these containers are two or more reaction containers. It is preferred that the two or more reaction containers should be connected to each other, from the viewpoint of handle ability. Examples of such reaction containers include a tube and a well plate (including a deep well plate). More specifically, examples thereof can include a 4-strip tube, an 8-strip tube (e.g., Figure 1), a 12-strip tube, a 24-well plate, a 48-well plate, a 96-well plate, and a 384-well plate. Among others, preferred examples thereof can include a reaction container made of plastic such as polystyrene or polypropylene. These tubes or well plates can be commercially available. The upper limit of the number of reaction containers in the kit of the present invention is not particularly limited and can be, for example, 384, 96, 48, 24, or 12.

Examples of a method for immobilizing the primer set for the target nucleic acid, the probe for the target nucleic acid, the positive control nucleic acid, the probe for the positive control nucleic acid, or other reagents described above into a reaction container can include, but are not particularly limited to, a method which involves adding each reagent into the container, and adding thereto a stabilizer such as trehalose, followed by drying. Among others, preferred examples thereof can include a method which involves drying under reduced pressure.

The reaction containers in the kit of the present invention preferably include a reaction container for a negative control, in addition to a reaction container for the test sample and a reaction container for the positive control nucleic acid. The primer set specific for the target nucleic acid, the probe specific for the target nucleic acid, and the probe specific for the positive control nucleic acid are immobilized in this reaction container for a negative control. For the detection or quantification of the target nucleic acid, no test sample is added to the reaction container for a negative control.

The reaction containers in the kit of the present invention preferably include an additional reaction container for the immobilization of the nucleic acid-polymerizing enzyme and/or the buffer for nucleic acid amplification reaction, in addition to a reaction container for the test sample and a reaction container for the positive control nucleic acid, and more preferably include at least an additional reaction container for the immobilization of the buffer for nucleic acid amplification reaction. This additional reaction container included therein may be connected to a reaction container for the test sample or a reaction container for the positive control nucleic acid or may be independent of these reaction containers. Figure 2 shows an example in which reaction container H for the immobilization of the buffer for nucleic acid amplification reaction is connected to reaction containers for the test sample and reaction containers for the positive control nucleic acid. Provided that a buffer necessary for the other reaction containers such as reaction containers A to G, preferably a slightly larger amount of the buffer for allowance (e.g., 103 to 120 parts by weight, preferably 105 to 120 parts by weight, of the buffer when the necessary amount of the buffer is defined as 100 parts by weight), is immobilized in the reaction container H, a buffer for real-time PCR can be immediately prepared by merely adding a predetermined amount of water to the reaction container H. In Figure 2, the buffer is required in an amount corresponding to 7 samples of the reaction containers A to G, and the buffer is immobilized in an amount corresponding to 7.5 samples in the reaction container H.

The two or more reaction containers in the kit of the present invention are preferably three or more reaction containers (preferably three or more reaction containers connected to each other), more preferably four or more reaction containers (preferably four or more reaction containers connected to each other).

When the kit of the present invention comprises three or more (preferably four or more) reaction containers or reaction containers connected to each other, it is preferred that: one or more (preferably 1 to 382, more preferably 1 to 381) reaction container (s) among the reaction containers should be a reaction container for the test sample; one or more (preferably two or more, more preferably 2 to 5, further preferably 2 to 4) reaction container(s) should be a reaction container for the positive control nucleic acid; and one or more (preferably one) reaction container(s) should be a reaction container for a negative control. In the case of using the kit of the present invention in the relative quantification of the target nucleic acid, one reaction container for the positive control nucleic acid may suffice. In the case of using the kit of the present invention in the absolute quantification of the target nucleic acid, it is preferred to provide two or more reaction containers for the positive control nucleic acid. The amount (concentration) of the positive control nucleic acid is gradually changed among the two or more reaction containers, and a more highly accurate calibration curve (regression equation) is obtained by the detection of signals of the probe for the target nucleic acid or the probe for the positive control nucleic acid. As a result, the absolute quantification of the target nucleic acid can be achieved more accurately.

In the case of providing two or more reaction containers for the positive control nucleic acid and changing the amount (concentration) of the positive control nucleic acid gradually among these containers, it is preferred that, when the reaction containers are ranked in an ascending order of the amount (concentration) of the positive control nucleic acid, the amount (concentration) should be increased, for example, in the range of 2 to 100 times, preferably in the range of 3 to 50 times, more preferably in the range of 4 to 20 times, further preferably in the range of 8 to 15 times, still further preferably 10 times, when lowered by one rank. In the case of changing the amount of the positive control nucleic acid gradually, the rate of increase may be the same among the stages or may be different among the stages. The amount (concentration) of the positive control nucleic acid in a reaction container having the largest amount of the positive control nucleic acid among the two or more reaction containers for the positive control nucleic acid mentioned above can be 10 or more times, preferably 20 or more times, more preferably 40 times, further preferably 100 or more times the amount (concentration) of the positive control nucleic acid in a reaction container having the smallest amount of the positive control nucleic acid.

### (Primer set specific for target nucleic acid according to present invention)

The kit of the present invention comprises the primer set specific for the target nucleic acid according to the present invention. The primer set specific for the target nucleic acid (primer set for the target nucleic acid) according to the present invention means a set of primers that specifically hybridize to a portion of the target nucleic acid and permit specific amplification of the target nucleic acid (hereinafter, also simply referred to as "primers for the target nucleic acid"). The set may comprise one or more sense primer(s) and one or more antisense primer(s) and preferably comprises one sense primer and one antisense primer with respect to one type of target nucleic acid, for use in an ordinary nucleic acid amplification method. However, depending on the nucleic acid amplification method used, such as LAMP or SMAP, one sense primer and one antisense primer per target nucleic acid are not enough, and two or more sense primers and/or antisense primers may be required. Therefore, those skilled in the art can select the constitution of the primer set according to the need. Each primer constituting the primer set is a polynucleotide. DNA is preferred from the viewpoint of stability. The number of nucleotides of each primer is not particularly limited as long as the target nucleic acid can be specifically amplified. Depending on the nucleic acid amplification method used, the number of nucleotides of each primer is usually in the range of 15 to 50, preferably in the range of 17 to 35. The primers in the set may have the same number of nucleotides or may have different numbers of nucleotides.

Those skilled in the art can appropriately select the nucleotide sequence of each primer in the primer set for the target nucleic acid, according to the target nucleic acid. Specifically, the nucleotide sequence of each primer for the target nucleic acid can be selected by examining the nucleotide sequences of regions near the target nucleic acid by use of, for example, sequence information of a sequence database, selecting a candidate sequence of the primer from among these nucleotide sequences, conducting homology search by BLAST or the like as to the candidate sequence, and further selecting a nucleotide sequence having low sequence identity to genomes except for the portion of the target nucleic acid, and transcripts thereof. In the specification of the present application, the 5' primer and the 3' primer may be a sense primer and an antisense primer, respectively, or may be an antisense primer and a sense primer, respectively.

The primer set for the target nucleic acid according to the present invention can be prepared by synthesis, nucleic acid amplification reaction, cloning, or a combination thereof according to a routine method and is preferably prepared by synthesis from the viewpoint of convenience. Such preparation of the primer set can also be commissioned to a company manufacturing oligonucleotide primers.

### (Probe specific for target nucleic acid according to present invention)

The kit of the present invention comprises the probe specific for the target nucleic acid according to the present invention. The probe specific for the target nucleic acid (probe for the target nucleic acid) according to the present invention means a probe that specifically hybridizes to a portion of the target nucleic acid and permits specific detection or quantification of the target nucleic acid. One probe for the target nucleic acid suffices for one target nucleic acid. Alternatively, two or more probes may be used for one target nucleic acid. The probe for the target nucleic acid is a polynucleotide such as DNA or RNA. DNA is preferred from the viewpoint of stability. The number of nucleotides of the probe for the target nucleic acid is not particularly limited as long as the probe for the target nucleic acid is capable of specifically hybridizing to the target nucleic acid. Depending on the nucleic acid amplification method used, the number of nucleotides of the probe for the target nucleic acid is preferably in the range of 9 to 100, more preferably in the range of 15 to 60, further preferably in the range of 20 to 40. The probe for the target nucleic acid may be double-stranded and is preferably single-stranded. A certain nucleotide sequence described in the present specification is mentioned as the sequence of DNA. RNA has a nucleotide sequence derived from the certain nucleotide sequence by the replacement of T with U.

It is preferred that the probe for the target nucleic acid according to the present invention should be labeled with a labeling material for the detection or quantification of an amplification product yielded by the corresponding primer pair. It is more preferred that the probe for the target nucleic acid should be labeled with a fluorescent material, from the viewpoint of more rapid or more highly sensitive detection or quantification. The site labeled with the labeling material other than the fluorescent material, and the labeling material other than the fluorescent material are as mentioned above. However, because the probe for the positive control nucleic acid and the probe for the target nucleic acid can be detected or quantified conveniently and rapidly at the same time, it is preferred that a label (preferably a fluorescent label) different from the label (preferably a fluorescent label) for the probe for the positive control nucleic acid should be used as the label (preferably a fluorescent label) for use in the probe for the target nucleic acid.

Examples of the type of the probe for the target nucleic acid according to the present invention include, but are not particularly limited to, TaqMan(R) probes, molecular beacon probes, cycling probes, Eprobe (R), Qprobe (R), scorpion probes, and hybridization-probes. Among others, a TaqMan probe is preferred. However, the type of the probe for the target nucleic acid according to the present invention is preferably the same as the type of the probe for the positive control nucleic acid according to the present invention, from the viewpoint of convenience, etc.

Those skilled in the art can appropriately select the nucleotide sequence of the probe for the target nucleic acid according to the present invention, according to the target nucleic acid. Such selection can be carried out by conducting homology search by BLAST or the like as to a candidate sequence of the probe selected from the nucleotide sequence of the target nucleic acid. When the target nucleic acid including the candidate sequence of the probe is selected from, for example, the genome of the organism species (e.g., a human) from which the test sample is derived, or a transcript thereof, a candidate sequence having low sequence identity to the "genome or a transcript thereof", except for the portion of the target nucleic acid, of at least the organism species can be selected as the nucleotide sequence of the probe for the target nucleic acid. On the other hand, when the target nucleic acid is selected from the genome of an organism species (e.g., a virus) other than the organism species (e.g., a human) from which the test sample is derived, or a transcript thereof, a candidate sequence having low sequence identity to the genome of at least the organism species (e.g., a human) from which the test sample is derived or a transcript thereof, and low sequence identity to the "genome or a transcript thereof", except for the portion of the target nucleic acid, of the organism species (e.g., a virus) from which the target nucleic acid is derived can be selected as the nucleotide sequence of the probe for the target nucleic acid.

The probe for the target nucleic acid according to the present invention can be prepared by synthesis, nucleic acid amplification reaction, cloning, or a combination thereof according to a routine method and is preferably prepared by synthesis from the viewpoint of convenience. Such preparation of the probe for the target nucleic acid can also be commissioned to a company manufacturing oligonucleotide probes.

The kit of the present invention may be a kit for use in the detection or quantification of two or more different types of target nucleic acids in a test sample. Such a kit is preferred because two or more different types of target nucleic acids in a test sample can be detected or quantified. The kit of the present invention that is used for such a purpose is not particularly limited as long as the kit comprises: two or more types of primer sets respectively specific for the two or more types of target nucleic acids; two or more types of probes respectively specific for the two or more types of target nucleic acids; a set comprising two or more types of positive control nucleic acids respectively serving as positive controls for the two or more types of target nucleic acids, and one or more (preferably one or two, more preferably one) types of probes specific for the two or more types of positive control nucleic acids; and two or more reaction containers, wherein
the two or more types of primer sets, the two or more types of probes, and the one or more (preferably one or two, more preferably one) types of probes are immobilized in reaction container(s) for the test sample, and the two or more types of primer sets, the two or more types of probes, the two or more types of positive control nucleic acids, and the one or more (preferably one or two, more preferably one) types of probes are immobilized in reaction container(s) for the positive control nucleic acids.

It is preferred that reagents, except for the primer set, necessary for the nucleic acid amplification reaction of the target nucleic acids should also be immobilized in a reaction container for the test sample and a reaction container for the positive control nucleic acids, from the viewpoint of more conveniently and rapidly detecting or quantifying the target nucleic acids.

The kit for use in the detection or quantification of two or more different types of target nucleic acids in a test sample is more preferably a kit wherein the two or more types of primer sets for the target nucleic acids, the two or more types of probes for the target nucleic acids, and the one or more (preferably one or two, more preferably one) types of probes for the positive control nucleic acids are immobilized in "each individual" reaction container for the test sample, and the two or more types of primer sets for the target nucleic acids, the two or more types of probes for the target nucleic acids, the two or more types of positive control nucleic acids, and the one or more (preferably one or two, more preferably one) types of probes for the positive control nucleic acids are immobilized in "each individual" reaction container for the positive control nucleic acids. Such combined use of the two or more types of primer sets, the two or more types of probes for the target nucleic acids, the one or more types of probes for the positive control nucleic acids, and the like in "each individual" reaction container for the test sample or "each individual" reaction container for the positive control nucleic acids is more preferred because two or more types of target nucleic acids in a test sample can be detected or quantified at the same time in fewer reaction containers while the presence or absence of contamination of a container for the test sample with a positive control nucleic acid is conveniently and rapidly confirmed.

The number of the type of the probe for the positive control nucleic acid contained in the kit of the present invention may be the same as the number of the type of the positive control nucleic acid contained in the kit of the present invention. From the viewpoint that more types of positive control nucleic acids are detected or quantified with fewer types of probes for the positive control nucleic acids, it is preferred to use at least one type of probe for the positive control nucleic acids that permits detection or quantification of two or more types of or all of the types of positive control nucleic acids among the positive control nucleic acids used in combination, and it is more preferred to use one type of probe for the positive control nucleic acids that permits detection or quantification of all of the types of positive control nucleic acids used in combination.

In the kit for use in the detection or quantification of two or more different types of target nucleic acids in a test sample, all of the two or more types of target nucleic acids may be nucleic acids of genes other than housekeeping gene. It is preferred that among the two or more types of target nucleic acids, at least one type of target nucleic acid should be a nucleic acid of a housekeeping gene, and at least one or more types of target nucleic acids should be nucleic acids of genes other than housekeeping gene. Use of a nucleic acid of a housekeeping gene as at least one type of target nucleic acid among the two or more types of target nucleic acids is useful in the measurement of the amounts of target nucleic acids with respect to a particular amount of the test sample or the correction of variations in the amount of the test sample among reaction containers, because the amount of a test sample (e.g., the number of test cells or the amount of genomic nucleic acids) in the test sample can be predicted.

### 3. [Method for detecting or quantifying target nucleic acid in test sample while confirming presence or absence of contamination of container for test sample with positive control nucleic acid]

The method for detecting or quantifying a target nucleic acid in a test sample while confirming the presence or absence of contamination of a container for the test sample with a positive control nucleic acid (hereinafter, also simply referred to as the "method of the present invention") is not particularly limited as long as the method comprises:
(a) step a of performing nucleic acid amplification reaction using a nucleic acid obtained from the test sample and a primer set specific for the target nucleic acid (primer set for the target nucleic acid of the present invention) in a reaction container for the test sample to obtain an amplification product;
(b) step b of performing nucleic acid amplification reaction using the positive control nucleic acid (positive control nucleic acid of the present invention) and the primer set specific for the target nucleic acid in a reaction container for the positive control nucleic acid to obtain an amplification product;
(c) step c of contacting the amplification product obtained in the step a with a probe specific for the target nucleic acid (probe for the target nucleic acid of the present invention) and a probe specific for the positive control nucleic acid (probe for the positive control nucleic acid of the present invention) in the reaction container for the test sample, and detecting or quantifying the presence or absence of the target nucleic acid and the positive control nucleic acid; and
(d) step d of contacting the amplification product obtained in the step b with the probe specific for the target nucleic acid and the probe specific for the positive control nucleic acid in the reaction container for the positive control nucleic acid, and detecting or quantifying the presence or absence of the target nucleic acid and the positive control nucleic acid, wherein
when the positive control nucleic acid is not detected in the step (c), it can be confirmed that the container for the test sample is not contaminated with the positive control nucleic acid.

The step a is not particularly limited as long as the step is of performing nucleic acid amplification reaction using a nucleic acid obtained from the test sample and a primer set for the target nucleic acid of the present invention in a reaction container for the test sample to obtain an amplification product. A method for obtaining the nucleic acid (preferably DNA or RNA) from the test sample is not particularly limited, and a routine method can be used. Specific examples of the method for obtaining DNA from the test sample can include "proteinase K/phenol extraction method", "proteinase K/phenol/chloroform extraction method", "alkaline dissolution method", "boiling method", and a method using a commercially available DNA extraction column. Specific examples of the method for obtaining RNA from the test sample can include "guanidine-cesium chloride ultracentrifugation method", "AGPC (acid guanidinium-phenol-chloroform) method", and a method using a commercially available RNA extraction column.

Examples of the type of the nucleic acid amplification reaction in the step a include, but are not particularly limited to, PCR (polymerase chain reaction) (preferably real-time PCR) and RT-PCR (reverse transcription polymerase chain reaction), and LCR (ligase chain reaction), which carry out temperature cycles, as well as various isothermal amplification methods, which involve no temperature cycles. Examples of the isothermal amplification methods include LAMP (loop-mediated isothermal amplification), SMAP (smart amplification process), NASBA (nucleic acid sequence-based amplification), ICAN (isothermal and chimeric primer-initiated amplification of nucleic acids)(R), TRC (transcription-reverse transcription concerted), SDA (strand displacement amplification), TMA (transcription-mediated amplification), and RCA (rolling circle amplification). These methods for nucleic acid amplification reaction are known in the art, and those skilled in the art can perform the nucleic acid amplification reaction, for example, by use of a commercially available kit, by appropriately setting nucleic acid amplification reaction conditions with reference to a literature known in the art or an instruction manual of the commercially available kit. The "nucleic acid amplification reaction" described in the present specification widely encompasses nucleic acid amplification reaction at varying temperatures or constant temperature with the aim of amplifying nucleic acids, in addition to those mentioned above.

In the nucleic acid amplification reaction in the step a, each reagent necessary for the nucleic acid amplification reaction can be used in addition to the nucleic acid obtained from the test sample and the primer set for the target nucleic acid of the present invention. Examples of such a reagent include one or more reagent(s) selected from the group consisting of a nucleic acid-polymerizing enzyme (e.g., polymerase), a substance serving as a material for nucleic acids (e.g., dNTP), a buffer for nucleic acid amplification reaction (e.g., a component having a buffering effect, such as Tris-Cl, and a surfactant such as Tween 20), and Mg²⁺. Other necessary reagents can be additionally used according to the type of the nucleic acid amplification reaction.

For the nucleic acid amplification reaction, a commercially available nucleic acid amplification apparatus can be used according to the type of the nucleic acid amplification reaction used, etc. Preferred examples of such an apparatus can include a nucleic acid amplification apparatus also equipped with an apparatus capable of measuring probe signals (preferably fluorescent signals). Examples of a real-time PCR apparatus capable of measuring both of nucleic acid amplification and fluorescent signals can include CFX96 manufactured by Bio-Rad Laboratories, Inc. and Light cycler 480 manufactured by F. Hoffmann-La Roche, Ltd.

The step b is not particularly limited as long as the step is of performing nucleic acid amplification reaction using the positive control nucleic acid of the present invention and the primer set specific for the target nucleic acid of the present invention in a reaction container for the positive control nucleic acid to obtain an amplification product.

The step c is not particularly limited as long as the step is of contacting the amplification product obtained in the step a with a probe specific for the target nucleic acid of the present invention and a probe specific for the positive control nucleic acid of the present invention in the reaction container for the test sample, and detecting or quantifying the presence or absence of the target nucleic acid and the positive control nucleic acid. For the detection or quantification of the presence or absence of the target nucleic acid or the detection or quantification of the presence or absence of the positive control nucleic acid, it is preferred to detect or quantify signals (preferably fluorescent signals) from the labeling material (preferably a fluorescent labeling material) on the probe for the target nucleic acid of the present invention or the labeling material (preferably a fluorescent labeling material) on the probe for the positive control nucleic acid of the present invention. Such detection or quantification is preferably performed using, for example, an apparatus capable of measuring fluorescent signals, included in a nucleic acid amplification apparatus. The obtained fluorescent signals can be analyzed using commercially available software such as Light cycler 480 software.

The step d is not particularly limited as long as the step is of contacting the amplification product obtained in the step b with the probe specific for the target nucleic acid and the probe specific for the positive control nucleic acid in the reaction container for the positive control nucleic acid, and detecting or quantifying the presence or absence of the target nucleic acid and the positive control nucleic acid. Such detection or quantification can be performed in the same way as in the detection or quantification in the step c.

In the method of the present invention, when the positive control nucleic acid is not detected in the step c, it can be confirmed that the container for the test sample is not contaminated with the positive control nucleic acid. The steps of the present invention may include, in addition to the steps a to d, the following step e:
(e) step e of examining whether the positive control nucleic acid is detected in the step c to thereby confirm the presence or absence of contamination of the container for the test sample with the positive control nucleic acid.

In the case of using the method of the present invention in the relative quantification of the target nucleic acid, one reaction container for the positive control nucleic acid suffices for use in the step b or the step d. In the case of using the method of the present invention in the absolute quantification of the target nucleic acid, it is preferred to use two or more reaction containers for the positive control nucleic acid in the step b or the step d. The amount (concentration) of the positive control nucleic acid is gradually changed among the two or more reaction containers, and a more highly accurate calibration curve (regression equation) is obtained by the detection of signals of the probe for the target nucleic acid or the probe for the positive control nucleic acid (preferably the probe for the target nucleic acid). As a result, the absolute quantification of the target nucleic acid can be achieved more accurately.

An approach known in the art, such as use of commercially available software, can be used as a method for calculating the calibration curve from the detected signals of the positive control nucleic acid. The method can preferably involve analyzing fluorescent signals by fit-point method or the like using, for example, Light cycler 480 software, and conducting regression analysis on the relationship between the Cq value (the number of reaction cycles required to reach particular signal intensity) of each target and the concentration of the positive control nucleic acid (preferably the copy number of the positive control nucleic acid) to thereby calculate the calibration curve (regression equation).

The intensity of signals (preferably fluorescent signals) of the probe for the target nucleic acid of the present invention measured in the step c can be applied to the obtained calibration curve to accurately determine the concentration of the target nucleic acid in the test sample.

In a preferred embodiment, examples of the method of the present invention can include a method for more conveniently and rapidly detecting or quantifying a target nucleic acid in a test sample by use of the kit of the present invention while conveniently and rapidly confirming the presence or absence of contamination of a container for the test sample with a positive control nucleic acid.

In the present specification, the "nucleotide sequence complementary to nucleotide sequence X" includes (X1) a nucleotide sequence that is derived from a nucleotide sequence strictly complementary to the nucleotide sequence X by the deletion, substitution, or addition of 1 or several (e.g., 1 to 5, preferably 1 to 3, more preferably 1 or 2, further preferably 1) nucleotides, and forms a specific hybrid (duplex molecule) with the nucleotide sequence X, and
(X2) a nucleotide sequence that has 95% or higher, preferably 97% or higher, more preferably 98% or higher, further preferably 99% or higher sequence identity to a nucleotide sequence strictly complementary to the nucleotide sequence X, and forms a specific hybrid with the nucleotide sequence X.
In the present specification, the term "strictly complementary" means "complementary" generally used. For example, a sequence strictly complementary to a DNA sequence of "ATGC" is "TACG".

In the present specification, the "nucleotide sequence specific for nucleotide sequence Y" includes (Y1) a nucleotide sequence that is derived from a nucleotide sequence strictly complementary to the nucleotide sequence Y by the deletion, substitution, or addition of 1 or several (e.g., 1 to 5, preferably 1 to 3, more preferably 1 or 2, further preferably 1) nucleotides, and forms a specific hybrid (duplex molecule) with the nucleotide sequence Y, and
(Y2) a nucleotide sequence that has 95% or higher, preferably 97% or higher, more preferably 98% or higher, further preferably 99% or higher sequence identity to a nucleotide sequence strictly complementary to the nucleotide sequence Y, and forms a specific hybrid with the nucleotide sequence Y.

Hereinafter, the present invention will be described in detail with reference to Examples. However, the present invention is not intended to be limited by these Examples.

### Example 1

### (Design of probe specific for positive control nucleic acid)

The nucleotide sequence of each probe specific for positive control nucleic acids (i.e., a nucleotide sequence complementary to nucleotide sequence C1 according to the present invention) was designed by a method as described below.
[1] The group consisting of repeat nucleotide sequences having 4 to 6 nucleotides obtained by repeating each nucleotide sequence having 2 nucleotides twice or three times was obtained. The "nucleotide sequence having 2 nucleotides" used was AT, AG, AC, TA, TG, TC, GA, GT, GC, CA, CT, and CG.
[2] Two or more repeat nucleotide sequences selected from the group obtained in the step [1] were linked at random to establish candidate nucleotide sequences.
[3] Whether each of the candidate nucleotide sequences established in the step [2] was a nucleotide sequence having 70% or lower sequence identity to the sequence of any portion of the genomes of 16000 organism species including an organism species (human) from which a test sample was derived and an organism species (EBV) from which a target nucleic acid was derived, and transcripts thereof was confirmed by use of, for example, sequence information of a sequence database (GenBank, etc.) as of May 2014 and software for homology search (BLAST(R), etc.). The BLAST homology search was conducted under the following conditions by selecting databases 1) to 3) in this order:
   Program: Nucleotide Blast
   Database: 1) Human genomic + transcript; 2) Mouse genomic + transcript; and 3) Others (nr etc.);
   Optimize for Highly similar sequence (Megablast)
[4] Whether the Tm values (°C) of the candidate nucleotide sequences established in the step [2] were 65°C or lower was confirmed by the nearest neighbor method.
[5] A candidate nucleotide sequence having 70% or lower sequence identity in the step [3] and having a Tm value of 65°C or lower in the step [4] was selected as the nucleotide sequence of each probe specific for positive control nucleic acids.

The nucleotide sequences of probes specific for positive control nucleic acids were actually selected as given below by the steps [1] to [5]. For the selection, the nucleotide sequences were selected such that the sequence identity in the step [3] and the temperature of the Tm value in the step [4] were as low as possible. CACA ATAT ACAC GCGC TATA TCTC ACAC (SEQ ID NO: 1) ATAT GAGA ACAC TGTG TCTC TATA GCGC (SEQ ID NO: 2) ATAT CACA ACAC TATA TCTC ACAC GCGC (SEQ ID NO: 3)

The nucleotide sequences of SEQ ID NOs: 1 to 3 each exhibited sequence identity in the homology search of the step [3] (highest value of the sequence identity in the homology search), and a Tm value (°C) as follows:

**[Table 1]**

| | Sequence identity | Tm value |
|---|---|---|
| SEQ ID NO: 1 | 60.7% | 44°C |
| SEQ ID NO: 2 | 67.9% | 44°C |
| SEQ ID NO: 3 | 64.0% | 42°C |

The probes respectively having the nucleotide sequences represented by SEQ ID NOs: 1 to 3 were prepared, and these probes were confirmed not to detect the genomic DNA of the following 13 organisms:
human, EBV, HSV1, HSV2, VZV, CMV, HHV6, HHV7, HHV8, PVB19 (parvovirus B19), JCV, HBV, and ADV1.

### Example 2

### (Preparation of positive control nucleic acid)

### 1. Preparation of positive control nucleic acid for EBV

A positive control nucleic acid for nucleotide positions 1889 to 2028 of EBV BALF5 gene (nucleotide sequence of nucleotide positions 153699 to 156746 of GenBank Accession No. V01555: SEQ ID NO: 4) as a target nucleic acid (hereinafter, referred to as "target nucleic acid EBV") (SEQ ID NO: 5) was prepared by a method as described below.

The nucleotide sequence of the target nucleic acid EBV was reported to Eurofins Genomics K.K. (formerly Operon Biotechnologies K.K.), and the synthesis of an artificial gene of the target nucleic acid EBV and an artificial gene of the positive control nucleic acid for EBV was commissioned to this company. The sequence (SEQ ID NO: 6) of the positive control nucleic acid for EBV was a sequence in which an insert sequence (nucleotide sequence complementary to the nucleotide sequence of SEQ ID NO: 2) complementary to the probe specific for positive control nucleic acids (SEQ ID NO: 2) prepared in Example 1 was inserted between nucleotide positions 99 and 100 of the sequence (SEQ ID NO: 5) of the target nucleic acid EBV. Each delivered artificial gene cloned in a vector was linearized. The amplification efficiency of the two artificial genes mentioned above was studied by comparison. The synthesis of a forward primer (5' primer) EBV-F (TAGGGCCAGTCAAAGTTG: SEQ ID NO: 7) and a reverse primer (3' primer) EBV-R (ACCTGCGAAGACATAGAG: SEQ ID NO: 8) capable of amplifying the target nucleic acid EBV, and EBV-probe (CGGTCACAATCTCCACGCTG: SEQ ID NO: 9) was commissioned to Nihon Techno Service Co., Ltd. Each of the two artificial genes mentioned above (the target nucleic acid EBV or the positive control nucleic acid for EBV) was used as a template in real-time PCR using both of the primers (EBV-F and EBV-R) and the probe (EBV-probe) to confirm that the positive control nucleic acid for EBV (SEQ ID NO: 6) was able to serve as an adequate positive control without difference in amplification efficiency between the artificial genes.

### 2. Preparation of positive control nucleic acid for human TBP gene

A positive control nucleic acid for nucleotide positions 170562136 to 170562215 of human TBP gene (NC_000006.12) as a target nucleic acid (hereinafter, referred to as "target nucleic acid TBP") (SEQ ID NO: 10) was prepared by a method as described below.

The nucleotide sequence of the target nucleic acid TBP was reported to Eurofins Genomics K.K. (formerly Operon Biotechnologies K.K.), and the synthesis of an artificial gene of the target nucleic acid TBP and an artificial gene of the positive control nucleic acid for TBP was commissioned to this company. The sequence (SEQ ID NO: 13) of the positive control nucleic acid for TBP was a sequence in which an insert sequence (nucleotide sequence complementary to the nucleotide sequence of SEQ ID NO: 2) complementary to the probe specific for positive control nucleic acids (SEQ ID NO: 2) prepared in Example 1 was inserted between nucleotide positions 29 and 30 of the sequence of the target nucleic acid TBP (SEQ ID NO: 10). Each delivered artificial gene cloned in a vector was linearized. The amplification efficiency of the two artificial genes mentioned above was studied by comparison. The synthesis of a forward primer (5' primer) TBP-F (GCACCACTCCACTGTATCCC: SEQ ID NO: 11) and a reverse primer (3' primer) TBP-R (CCCAGAACTCTCCGAAGCTG: SEQ ID NO: 12) capable of amplifying the target nucleic acid TBP, and TBP-probe (ACCCCCATCACTCCTGCCACGC: SEQ ID NO: 14) was commissioned to Nihon Techno Service Co., Ltd. Each of the two artificial genes mentioned above (the target nucleic acid TBP or the positive control nucleic acid for TBP) was used as a template in real-time PCR using both of the primers (TBP-F and TBP-R) and the probe (TBP-probe) to confirm that the positive control nucleic acid for TBP (SEQ ID NO: 13) was able to serve as an adequate positive control without difference in amplification efficiency between the artificial genes.

### Example 3

### (Preparation of kit for use in detection or quantification of target nucleic acid in test sample)

A kit for use in the detection or quantification of target nucleic acids in a test sample was prepared by a method as described below.

First, a commercially available 8-strip PCR tube (manufactured by F. Hoffmann-La Roche, Ltd.) was prepared. This tube is constituted by 8 reaction containers (wells) connected in a line (see Figure 1). EBV-F, EBV-R, and EBV-probe mentioned above were prepared as an EBV primer-probe mix (EBV PPmix) capable of specifically amplifying or detecting the target nucleic acid EBV. TBP-F, TBP-R, and TBP-probe mentioned above were prepared as TBP PPmix capable of specifically amplifying the target nucleic acid TBP. In Example 2, the probe of SEQ ID NO: 2 was used as the probe specific for positive control nucleic acids. In this Example 3, IC-probe (CACATGTGATATGCGCAGAGTCTC: SEQ ID NO: 15) was prepared. A new positive control nucleic acid for EBV (SEQ ID NO: 16) and a new positive control nucleic acid for TBP gene (SEQ ID NO: 17) were prepared in the same way as in Example 2. These positive control nucleic acids were designed so as to hybridize to the "probe specific for positive control nucleic acids (IC-probe)" of SEQ ID NO: 15, not the "probe specific for positive control nucleic acids" of SEQ ID NO: 2. Specifically, the new positive control nucleic acid for EBV (SEQ ID NO: 16) was a sequence in which an insert sequence complementary to IC-probe (SEQ ID NO: 15) was inserted between nucleotide positions 99 and 100 of the sequence (SEQ ID NO: 5) of the target nucleic acid EBV. The new positive control nucleic acid for TBP gene (SEQ ID NO: 17) was a sequence in which an insert sequence (nucleotide sequence complementary to the nucleotide sequence of SEQ ID NO: 2) complementary to IC-probe (SEQ ID NO: 15) was inserted between nucleotide positions 29 and 30 of the sequence of the target nucleic acid TBP (SEQ ID NO: 10).

The nucleotide sequence (SEQ ID NO: 9) of EBV-probe mentioned above is a nucleotide sequence corresponding to a nucleotide sequence of nucleotide positions 32 to 51 of the target nucleic acid EBV (SEQ ID NO: 5). The EBV-probe was modified at its 5' end with a reporter fluorescent dye 6-FAM and at its 3' end with a quencher fluorescent dye BHQ-1. The nucleotide sequence (SEQ ID NO: 14) of TBP-probe mentioned above is a nucleotide sequence corresponding to a nucleotide sequence of nucleotide positions 40 to 61 of the target nucleic acid TBP (SEQ ID NO: 10). The TBP-probe was modified at its 5' end with a reporter fluorescent dye HEX and at its 3' end with a quencher fluorescent dye BHQ-1. The nucleotide sequence (SEQ ID NO: 15) of IC-probe mentioned above is a nucleotide sequence complementary to a nucleotide sequence of nucleotide positions 100 to 123 of the positive control nucleic acid for EBV (SEQ ID NO: 6), and is a nucleotide sequence complementary to a nucleotide sequence of nucleotide positions 30 to 53 of the positive control nucleic acid for TBP gene (SEQ ID NO: 13). The IC-probe was modified at its 5' end with a reporter fluorescent dye Cy5 and at its 3' end with a quencher fluorescent dye BHQ-3.

As shown in Figure 1, among 8 reaction containers (A to H in order from the left) of the 8-strip PCR tube mentioned above, 3 reaction containers (A to C) were used as reaction containers for the positive control nucleic acids, 4 reaction containers (D to G) were used as reaction containers for the test sample, and the reaction container H was used as a reaction container for a negative control.

To each of the reaction containers A to C for the positive control nucleic acids, EBV PPmix (EBV-F, EBV-R, and EBV-probe), TBP PPmix (TBP-F, TBP-R, and TBP-probe), IC-probe, the positive control nucleic acid for EBV, and the positive control nucleic acid for TBP gene were added, and then a stabilizer such as trehalose was further added. Subsequently, these reagents were immobilized by drying under reduced pressure. The concentration of each positive control nucleic acid was gradually changed among the reaction containers A to C, and was set to 1 × 10¹ copies for the reaction container C, 1 × 10³ copies for the reaction container B, and 1 × 10⁵ copies for the reaction container A.

To each of the reaction containers D to G for the test sample, EBV PPmix (EBV-F, EBV-R, and EBV-probe), TBP PPmix (TBP-F, TBP-R, and TBP-probe), and IC-probe were added, and then a stabilizer such as trehalose was further added. Subsequently, these reagents were immobilized by drying under reduced pressure.

To the reaction container H for a negative control, EBV PPmix (EBV-F, EBV-R, and EBV-probe), TBP PPmix (TBP-F, TBP-R, and TBP-probe), and IC-probe were added, and then a stabilizer such as trehalose was further added, as with the reaction containers D to G. Subsequently, these reagents were immobilized by drying under reduced pressure.

In this way, various reagents were immobilized in each reaction container of the 8-strip PCR tube to prepare an 8-strip PCR tube for use in the detection or quantification of target nucleic acids in a test sample.

### Example 4

### (Real-time PCR assay using reagent-immobilized 8-strip PCR tube - 1)

The reagent-immobilized 8-strip PCR tube prepared in Example 3 was used to conduct the multiplex real-time PCR assay of the EBV BALF5 gene and the human TBP gene by a method as described below.

Genomic DNA extracted from an EBV virus-positive cell line SNT8 was prepared as a simulated test sample. Next, 10 µL of Premix EX Taq (probe qPCR) manufactured by Takara Bio Inc. was added to each reaction container of the reagent-immobilized 8-strip PCR tube. Premix EX Taq contains Taq polymerase TaKaRa Ex Taq HS, dNTP Mixture, Mg²⁺, and buffer components. No simulated sample was added to the reaction containers A to C for the positive control nucleic acids and the reaction container H for a negative control. 100 ng, 10 ng, 1 ng, and 0.1 ng of the simulated test sample genomic DNA were added to the reaction containers D, E, F, and G for the test sample, respectively. The reaction solution in each reaction container was adjusted to 20 µL.

The real-time PCR apparatus used was CFX96 manufactured by Bio-Rad Laboratories, Inc. The PCR reaction conditions involved heat treatment at 95°C for 10 seconds, followed by 45 reaction cycles each consisting of 95°C for 5 seconds and 60°C for 30 seconds. The amounts of PCR amplification products were determined by the detection or quantification of the fluorescence of 6-FAM on EBV-probe, the fluorescence of HEX on TBP-probe, and the fluorescence of Cy5 on IC-probe. These 3 fluorescent signals were analyzed by the fit-point method using Light cycler 480 software to determine the Cq value (the number of reaction cycles required to reach particular signal intensity) of each target.

### [Results about reaction containers A to C]

The results of the real-time PCR assay mentioned above about the reaction containers A to C (reaction containers for the positive control nucleic acids) are shown in Figure 3. In all of the reaction containers A to C, the fluorescent signals of the 3 types of probes (EBV-probe (6-FAM), TBP-probe (HEX), and IC-probe (Cy5)) were detected. Furthermore, the fluorescent signals were detected even at a smaller number of reaction cycles in a reaction container containing a larger copy number of the positive control nucleic acids among the reaction container A (positive control nucleic acid: 1 × 10⁵ copies), the reaction container B (positive control nucleic acid: 1 × 10³ copies), and the reaction container C (positive control nucleic acid: 1 × 10¹ copies).

For each of the reaction containers A to C, regression analysis was conducted using the calculated Cq value (Y) of each target and the numerical value of the copy number of each positive control nucleic acid indicated in common logarithm (X) to determine a regression equation. The results are shown in Figure 4. As shown in Figure 4, all of the regression equation for the target EBV, the regression equation for the target TBP, and the regression equation for the target IC were primary expressions which were linear. In addition, their determination coefficients R² (square of correlation coefficient R) were very high. These results demonstrated that the set of the present invention comprising a positive control nucleic acid and a probe for the positive control nucleic acid, and the kit of the present invention such as a reagent-immobilized 8-strip PCR tube are excellent in the quantitative performance of detection and produce a calibration curve excellent in quantitative performance.

The regression equation for the target EBV was Y = - 3.1483X + 34.623 with determination coefficient R² = 1. The regression equation for the target TBP was Y = -3.3332 + 35.702 with determination coefficient R² = 0.99999. The regression equation for the target IC was Y = -3.175 + 34.089 with determination coefficient R² = 0.99998.

### [Results about reaction containers D to G]

The results of the real-time PCR assay mentioned above about the reaction containers D to G (reaction containers for the test sample) are shown in Figure 5. In all of the reaction containers D to G, the fluorescent signals of two types of probes (EBV-probe (6-FAM) and TBP-probe (HEX)) were detected, whereas the fluorescent signals of IC-probe (Cy5) were not detected. Unlike the positive control nucleic acids, the simulated test sample genomic DNA mentioned above was free from a sequence hybridizable with the IC-probe (IC sequence). The fluorescent signals of the IC-probe were not detected in the reaction containers D to G, indicating that the IC-probe did not nonspecifically hybridize to the human or virus genome or transcripts thereof. Thus, if the fluorescent signals of the IC-probe are detected in a reaction container for the test sample, the reaction container for the test sample is confirmed to be contaminated by a positive control nucleic acid. The fluorescent signals of EBV-probe (6-FAM) and TBP-probe (HEX) were detected even at a smaller number of reaction cycles in a reaction container containing a larger amount of the simulated test sample added among the reaction container D (simulated test sample: 100 ng), the reaction container E (simulated test sample: 10 ng), the reaction container F (simulated test sample: 1 ng), and the reaction container G (simulated test sample: 0.1 ng)

### [Results about reaction container H]

As a result of the real-time PCR assay mentioned above, none of the fluorescent signals of the three types of probes (EBV-probe (6-FAM), TBP-probe (HEX), and IC-probe (Cy5)) were detected in the reaction container H (reaction container for a negative control). This is a natural outcome because the reaction container H contained neither of the positive control nucleic acids nor the simulated test sample.

### Example 5

### (Real-time PCR assay using reagent-immobilized 8-strip PCR tube - 2)

The following real-time PCR assay was conducted in order to examine detection results produced when a reaction container for the test sample was contaminated with even 1 copy of a positive control nucleic acid.

The real-time PCR assay was conducted in the same way as the method of Example 4 except that no simulated test sample was added to the reaction container D (reaction container for the test sample) of the reagent-immobilized 8-strip PCR tube, and the reaction container was contaminated with 1 copy of the positive control nucleic acid for EBV.

The results of the real-time PCR assay mentioned above about the reaction container D are shown in Figure 6. The fluorescent signals of two types of probes (EBV-probe (6-FAM) and IC-probe (Cy5)) were also detected in the reaction container D. These results demonstrated that the set of the present invention comprising a positive control nucleic acid and a probe for the positive control nucleic acid, and the kit of the present invention such as a reagent-immobilized 8-strip PCR tube can detect even the 1-copy level of contamination with a positive control nucleic acid. Since the reaction container D in this real-time PCR assay was free from the human genome contained in the simulated test sample, the fluorescent signals of TBP-probe (HEX) were not detected.

### Example 6

### (Real-time PCR assay using reagent-immobilized 8-strip PCR tube - 3)

In Examples 4 and 5, the primers, the probes, and the positive control nucleic acids were immobilized in the 8-strip PCR tube, whereas neither the polymerase enzyme nor the buffer was immobilized. The following real-time PCR assay was conducted in order to examine whether to influence preservation stability at room temperature when the primers, the probes, and the positive control nucleic acids as well as the polymerase enzyme and the buffer were immobilized in the same tube.

Reaction containers A and B of a commercially available 8-strip PCR tube (manufactured by F. Hoffmann-La Roche, Ltd.) were used as reaction containers for the positive control nucleic acids in the same way as in Example 3 except that the concentration of each positive control nucleic acid was the same between the reaction containers A and B. EBV PPmix (EBV-F, EBV-R, and EBV-probe), TBP PPmix (TBP-F, TBP-R, and TBP-probe), IC-probe, the positive control nucleic acid for EBV, and the positive control nucleic acid for TBP gene (hereinafter, also referred to as "PPmix, etc."), and Taq polymerase TaKaRa Ex Taq Hot Start Version (manufactured by Takara Bio Inc.) were immobilized in the reaction container B. A buffer for reaction container B (also containing Mg²⁺ and dNTP) was immobilized in the reaction container B as well as reaction container C. On the other hand, PPmix, etc. and TaKaRa Ex Taq Hot Start Version (manufactured by Takara Bio Inc.) as well as a buffer (also containing Mg²⁺ and dNTP) was immobilized in the reaction container A. This 8-strip PCR tube was preserved at room temperature for 2 months. Then, real-time PCR was conducted in the same way as the method of Example 4 except that TaKaRa Ex Taq Hot Start Version was used as Taq polymerase instead of TaKaRa Ex Taq HS.

The results of the real-time PCR assay mentioned above about the reaction container A (PPmix, etc., the enzyme, and the buffer were immobilized in one reaction container) are shown in the left graph of Figure 7, and the results thereof about the reaction container B (PPmix, etc. and the enzyme were immobilized in one container, and the buffer was immobilized in another container) are shown in the right graph of Figure 7. As is evident from Figure 7, the fluorescence of the 3 labels was properly confirmed in the reaction container B (PPmix, etc. and the enzyme were immobilized in one container, and the buffer was immobilized in another container) with the highest fluorescence intensity (RFU) detected, whereas the fluorescence of one of the 3 labels was unable to be confirmed in the reaction container A (PPmix, etc., the enzyme, and the buffer were immobilized in one reaction container) with lower fluorescence intensity of the other labels than that of the reaction container B. These results demonstrated that when PPmix, etc., the enzyme, and the buffer are immobilized in one reaction container, long-term preservation stability at room temperature may be reduced so that detection sensitivity may be slightly reduced in real-time PCR. Thus, it is suggested that, from the viewpoint of obtaining long-term preservation stability at room temperature, the enzyme and the buffer (also containing Mg²⁺ and dNTP) are preferably immobilized in another reaction container. Even when PPmix, etc., the enzyme, and the buffer were immobilized in one reaction container, a preservation period on the order of 2 weeks at room temperature did not present particular problems with detection sensitivity, and a preservation period on the order of 1 week did not present any problem with detection sensitivity.

### Industrial Applicability

The present invention can provide a set capable of conveniently and rapidly confirming the presence or absence of contamination of a container for a test sample, the set comprising a positive control nucleic acid and a probe specific for the positive control nucleic acid. The present invention can also provide a method for detecting or quantifying a target nucleic acid in a test sample while conveniently and rapidly confirming the presence or absence of contamination of a container for the test sample with a positive control nucleic acid.

## Claims

1. A set for use for at least confirming the presence or absence of contamination of a container for a test sample with a positive control nucleic acid in the detection or quantification of a target nucleic acid in a test sample using a primer set specific for the target nucleic acid and a probe specific for the target nucleic acid, the set comprising a positive control nucleic acid and a probe specific for the positive control nucleic acid,
wherein the positive control nucleic acid serves as a positive control of the target nucleic acid and is any one of a nucleic acid selected from (I) a single-stranded nucleic acid consisting of a nucleotide sequence having the following features (A) to (D), (II) a single-stranded nucleic acid consisting of a nucleotide sequence complementary to the nucleotide sequence of the single-stranded nucleic acid (I), and (III) a double-stranded nucleic acid formed from the single-stranded nucleic acid (I) and the single-stranded nucleic acid (II),
wherein the positive control nucleic acid can be amplified using the primer set specific for the target nucleic acid,
wherein the nucleotide sequence of the probe specific for the positive control nucleic acid is a nucleotide sequence that has 70% or lower sequence identity to the sequence of any portion of the genomes of an organism species from which the test sample is derived and an organism species from which the target nucleic acid is derived, and transcripts thereof, and has a Tm value of 65°C or lower; and
wherein the nucleotide sequence of the probe specific for the positive control nucleic acid is a nucleotide sequence that has 70% or lower sequence identity to the sequence of any portion of the genomes of 20 or more species selected from the group consisting of mammals, bacteria, fungi and viruses, and transcripts thereof, and has a Tm value of 65°C or lower:
(A) comprising a nucleotide sequence A1 of a 5' primer specific for the target nucleic acid and a nucleotide sequence A2 complementary to a 3' primer specific for the target nucleic acid;
(B) comprising a nucleotide sequence B1 complementary to the probe specific for the target nucleic acid, or a nucleotide sequence B2 complementary to the nucleotide sequence B1;
(C) comprising a nucleotide sequence C1 complementary to the probe specific for the positive control nucleic acid, or a nucleotide sequence C2 complementary to the nucleotide sequence C1; and
(D) the nucleotide sequence B1 or B2 and the nucleotide sequence C1 or C2 are positioned between the nucleotide sequences A1 and A2.

2. The set according to claim 1, wherein the nucleotide sequence (I) in the nucleotide sequence of the positive control nucleic acid is
(I') a nucleotide sequence wherein the nucleotide sequence C1 or C2 is inserted to a portion other than the nucleotide sequences A1 and A2 and the nucleotide sequence B1 or B2 in a single-stranded nucleic acid having the nucleotide sequences A1 and A2 and the nucleotide sequence B1 or B2 in a nucleotide sequence derived from the nucleotide sequence of the target nucleic acid, or
a nucleotide sequence wherein one or more nucleotide sequence(s) of a portion other than the nucleotide sequences A1 and A2 and the nucleotide sequence B1 or B2 is substituted with the nucleotide sequence C1 or C2 in a single-stranded nucleic acid having the nucleotide sequences A1 and A2 and the nucleotide sequence B1 or B2 in a nucleotide sequence derived from the nucleotide sequence of the target nucleic acid.

3. The set according to claim 1 or 2, wherein the number of nucleotides of the nucleotide sequences A1 and A2 is within the range of 15 to 50, respectively, the number of nucleotides of the nucleotide sequences B1 and B2 is within the range of 15 to 100, respectively, the number of nucleotides of the nucleotide sequences C1 and C2 is within the range of 15 to 100, respectively, and the number of nucleotides of the probe specific for the positive control nucleic acid is within the range of 15 to 100.

4. The set according to any one of claims 1 to 3, wherein the nucleotide sequence of the probe specific for the positive control nucleic acid is a nucleotide sequence having a linkage of two or more nucleotide sequences, which is selected from the group consisting of repeat nucleotide sequences having 4 to 8 nucleotides obtained by repeating twice to four times any of the following nucleotide sequences having 2 nucleotides:
AT, AG, AC, TA, TG, TC, GA, GT, GC, CA, CT, and CG.

5. A kit for use in the detection or quantification of a target nucleic acid in a test sample, the kit comprising: a primer set specific for the target nucleic acid; a probe specific for the target nucleic acid; a set according to any one of claims 1 to 4 comprising a positive control nucleic acid and a probe specific for the positive control nucleic acid; and two or more reaction containers, wherein
the primer set specific for the target nucleic acid, the probe specific for the target nucleic acid, and the probe specific for the positive control nucleic acid are immobilized in at least one of the reaction container(s) for the test sample among the two or more reaction containers, and the primer set specific for the target nucleic acid, the probe specific for the target nucleic acid, the positive control nucleic acid, and the probe specific for the positive control nucleic acid are immobilized in at least one of the remaining reaction container(s) for the positive control nucleic acid.

6. The kit according to claim 5 for use in the detection or quantification of two different types of target nucleic acids in a test sample, wherein
the kit comprises: two types of primer sets each specific for the two types of target nucleic acids; two types of probes each specific for the two types of target nucleic acids; a set comprising two types of positive control nucleic acids each serving as a positive control for the two types of target nucleic acids, and one or two types of probes specific for the two types of positive control nucleic acids; and two or more reaction containers, wherein
among the two types of target nucleic acids, one type of target nucleic acid is a nucleic acid of a gene other than housekeeping gene, and the other type of target nucleic acid is a nucleic acid of a housekeeping gene,
the two types of primer sets, the two types of probes, and the one or two types of probes are immobilized in each individual reaction container for the test sample, and the two types of primer sets, the two types of probes, the two types of positive control nucleic acids, and the one or two types of probes are immobilized in each of the reaction container for the positive control nucleic acids.

7. The kit according to claim 5 or 6, wherein polymerase is further immobilized in each reaction container.

8. The kit according to claim 6 or 7, wherein
the two or more reaction containers are three or more reaction containers connected to each other,
among the reaction containers, one or more reaction container(s) is a reaction container for the test sample, one or more reaction container(s) is a reaction container for the positive control nucleic acids, and one or more reaction container(s) is a reaction container for a negative control, and
wherein the two types of primer sets each specific for the two types of target nucleic acids, the two types of probes each specific for the two types of target nucleic acids, and one or two types of probes specific for the two types of positive control nucleic acids are immobilized in the reaction container for a negative control.

9. The kit according to claim 8, wherein
the three or more reaction containers connected to each other are four or more reaction containers connected to each other,
two or more reaction containers are reaction containers for the positive control nucleic acids among the reaction containers,
the amount of the positive control nucleic acid immobilized in the two or more reaction containers gradually differs, and the amount of the positive control nucleic acid in a reaction container having the largest amount of the positive control nucleic acid among the two or more reaction containers is 10 or more times the amount of the positive control nucleic acid in a reaction container having the smallest amount of the positive control nucleic acid.

10. A method for detecting or quantifying a target nucleic acid in a test sample while confirming the presence or absence of contamination of a container for the test sample with a positive control nucleic acid, the method comprising:
(a) step "a" of performing a nucleic acid amplification reaction using a nucleic acid obtained from the test sample and a primer set specific for the target nucleic acid in a reaction container for the test sample to obtain an amplification product;
(b) step "b" of performing a nucleic acid amplification reaction using the positive control nucleic acid and the primer set specific for the target nucleic acid in a reaction container for the positive control nucleic acid to obtain an amplification product;
(c) step "c" of contacting the amplification product obtained in the step "a" with a probe specific for the target nucleic acid and a probe specific for the positive control nucleic acid in the reaction container for the test sample, and detecting or quantifying the presence or absence of the target nucleic acid and the positive control nucleic acid; and
(d) step "d" of contacting the amplification product obtained in the step "b" with the probe specific for the target nucleic acid and the probe specific for the positive control nucleic acid in the reaction container for the positive control nucleic acid, and detecting or quantifying the presence or absence of the target nucleic acid and the positive control nucleic acid, wherein
when the positive control nucleic acid is not detected in the step "c", it can be confirmed that the container for the test sample is not contaminated with the positive control nucleic acid,
the positive control nucleic acid serves as a positive control of the target nucleic acid and is any one of a nucleic acid selected from (I) a single-stranded nucleic acid consisting of a nucleotide sequence having the following features (A) to (D), (II) a single-stranded nucleic acid consisting of a nucleotide sequence complementary to the nucleotide sequence of the single-stranded nucleic acid (I), and (III) a doublestranded nucleic acid formed from the single-stranded nucleic acid (I) and the single-stranded nucleic acid (II),
wherein the positive control nucleic acid can be amplified using the primer set specific for the target nucleic acid,
wherein the nucleotide sequence of the probe specific for the positive control nucleic acid is a nucleotide sequence that has 70% or lower sequence identity to the sequence of any portion of the genomes of an organism species from which the test sample is derived and an organism species from which the target nucleic acid is derived, and transcripts thereof, and has a Tm value of 65°C or lower; and
wherein the nucleotide sequence of the probe specific for the positive control nucleic acid is a nucleotide sequence that has 70% or lower sequence identity to the sequence of any portion of the genomes of 20 or more species selected from the group consisting of mammals, bacteria, fungi and viruses, and transcripts thereof, and has a Tm value of 65°C or lower:
(A) comprising a nucleotide sequence A1 of a 5' primer specific for the target nucleic acid and a nucleotide sequence A2 complementary to a 3' primer specific for the target nucleic acid;
(B) comprising a nucleotide sequence B1 complementary to the probe specific for the target nucleic acid, or a nucleotide sequence B2 complementary to the nucleotide sequence Bi;
(C) comprising a nucleotide sequence C1 complementary to the probe specific for the positive control nucleic acid, or a nucleotide sequence C2 complementary to the nucleotide sequence C1; and
(D) the nucleotide sequence B1 or B2 and the nucleotide sequence C1 or C2 are positioned between the nucleotide sequences A1 and A2.

11. The method according to claim 10 for detecting or quantifying two different types of target nucleic acids in a test sample while confirming the presence or absence of contamination of a container for the test sample with a positive control nucleic acid, the method comprising
using two types of primer sets each specific for the two types of target nucleic acids as the primer set specific for the target nucleic acid,
using two types of positive control nucleic acids as the positive control nucleic acid,
using two types of probes each specific for the two types of target nucleic acids as the probe specific for the target nucleic acid,
using one or two types of probes specific for the two types of positive control nucleic acids as the probe specific for the positive control nucleic acid, and
detecting or quantifying the presence or absence of the two types of target nucleic acids and the two types of positive control nucleic acids, wherein
when the positive control nucleic acid is not detected in the step "c", it can be confirmed that the container for the test sample is not contaminated with the positive control nucleic acid, and
among the two types of target nucleic acids, one type of target nucleic acid is a nucleic acid of a gene other than housekeeping gene, and the other type of target nucleic acid is a nucleic acid of a housekeeping gene.

## Patentansprüche

1. Satz zur Verwendung für zumindest das Bestätigen des Vorhandenseins oder der Abwesenheit einer Kontamination eines Behälters für eine Testprobe mit einer Positivkontrollnukleinsäure beim Nachweis oder der Quantifizierung einer Zielnukleinsäure in einer Testprobe unter Verwendung eines für die Zielnukleinsäure spezifischen Primersatzes und einer für die Zielnukleinsäure spezifischen Sonde, wobei der Satz eine Positivkontrollnukleinsäure und eine für die Positivkontrollnukleinsäure spezifische Sonde umfasst,
wobei die Positivkontrollnukleinsäure als eine Positivkontrolle der Zielnukleinsäure dient und eine von einer Nukleinsäure ist, die ausgewählt ist aus (I) einer einzelsträngigen Nukleinsäure, die aus einer Nukleotidsequenz mit den folgenden Merkmalen (A) bis (D) besteht, (II) einer einzelsträngigen Nukleinsäure, die aus einer Nukleotidsequenz besteht, die zur Nukleotidsequenz der einzelsträngigen Nukleinsäure (I) komplementär ist, und (III) einer doppelsträngigen Nukleinsäure, die aus der einzelsträngigen Nukleinsäure (I) und der einzelsträngigen Nukleinsäure (II) gebildet wird,
wobei die Positivkontrollnukleinsäure unter Verwendung des Primersatzes, der für die Zielnukleinsäure spezifisch ist, amplifiziert werden kann,
wobei die Nukleotidsequenz der Sonde, die für die Positivkontrollnukleinsäure spezifisch ist, eine Nukleotidsequenz ist, die eine Sequenzidentität von 70 % oder niedriger zur Sequenz eines beliebigen Teils der Genome einer Organismusspezies, von der die Testprobe stammt, und einer Organismusspezies aufweist, von der die Zielnukleinsäure und die Transkripte davon stammen, und die einen Tm-Wert von 65 °C oder niedriger hat; und
wobei die Nukleotidsequenz der Sonde, die für die positive Kontrollnukleinsäure spezifisch ist, eine Nukleotidsequenz ist, die eine Sequenzidentität von 70 % oder niedriger zur Sequenz eines beliebigen Teils der Genome von 20 oder mehr Spezies aufweist, die aus der Gruppe ausgewählt ist, die aus Säugetieren, Bakterien, Pilzen und Viren und den Transkripten davon besteht, und die einen Tm-Wert von 65 °C oder niedriger hat:
(A) wobei die eine Nukleotidsequenz A1 eines 5'-Primers, der für die Zielnukleinsäure spezifisch ist, und eine Nukleotidsequenz A2 umfasst, die zu einem 3'-Primer komplementär ist, der für die Zielnukleinsäure spezifisch ist;
(B) wobei die eine Nukleotidsequenz B1, die zur Sonde komplementär ist, die für die Zielnukleinsäure spezifisch ist, oder eine Nukleotidsequenz B2 umfasst, die zur Nukleotidsequenz B1 komplementär ist;
(C) wobei die eine Nukleotidsequenz C1, die zur Sonde komplementär ist, die für die Positivkontrollnukleinsäure spezifisch ist, oder eine Nukleotidsequenz C2 umfasst, die zur Nukleotidsequenz C1 komplementär ist; und
(D) wobei die Nukleotidsequenz B1 oder B2 und die Nukleotidsequenz C1 oder C2 zwischen den Nukleotidsequenzen A1 und A2 positioniert sind.

2. Satz gemäß Anspruch 1, wobei die Nukleotidsequenz (I) in der Nukleotidsequenz der Positivkontrollnukleinsäure ist
(I') eine Nukleotidsequenz, wobei die Nukleotidsequenz C1 oder C2 in einen anderen Teil als die Nukleotidsequenzen A1 und A2 und die Nukleotidsequenz B1 oder B2 in einer einzelsträngigen Nukleinsäure mit den Nukleotidsequenzen A1 und A2 und der Nukleotidsequenz B1 oder B2 in einer Nukleotidsequenz eingefügt ist, die von der Nukleotidsequenz der Zielnukleinsäure abgeleitet ist, oder
eine Nukleotidsequenz, wobei eine oder mehrere Nukleotidsequenz(en) eines anderen Teils als der Nukleotidsequenzen A1 und A2 und der Nukleotidsequenz B1 oder B2 in einer einzelsträngigen Nukleinsäure mit den Nukleotidsequenzen A1 und A2 und der Nukleotidsequenz B1 oder B2 durch die Nukleotidsequenz C1 oder C2 in einer Nukleotidsequenz substituiert sind, die von der Nukleotidsequenz der Zielnukleinsäure stammt.

3. Satz gemäß Anspruch 1 oder 2, wobei die Anzahl der Nukleotide der Nukleotidsequenzen A1 und A2 entsprechend im Bereich von 15 bis 50 liegt, wobei die Anzahl der Nukleotide der Nukleotidsequenzen B1 und B2 entsprechend im Bereich von 15 bis 100 liegt, wobei die Anzahl der Nukleotide der Nukleotidsequenzen C1 und C2 im Bereich von 15 bis 100 liegt, und die Anzahl der Nukleotide der Sonde, die für die Positivkontrollnukleinsäure spezifisch sind, im Bereich von 15 bis 100 liegt.

4. Satz gemäß einem der Ansprüche 1 bis 3, wobei die Nukleotidsequenz der Sonde, die für die Positivkontrollnukleinsäure spezifisch ist, eine Nukleotidsequenz mit einer Verknüpfung von zwei oder mehr Nukleotidsequenzen ist, die aus der Gruppe ausgewählt ist, die aus wiederholten Nukleotidsequenzen mit 4 bis 8 Nukleotiden besteht, die durch zweimaliges bis viermaliges Wiederholen einer der folgenden Nukleotidsequenzen mit 2 Nukleotiden erhalten wird:
AT, AG, AC, TA, TG, TC, GA, GT, GC, CA, CT und CG.

5. Kit zur Verwendung beim Nachweis oder zur Quantifizierung einer Zielnukleinsäure in einer Testprobe, wobei das Kit Folgendes umfasst: einen Primersatz, der für die Zielnukleinsäure spezifisch ist; eine Sonde, die für die Zielnukleinsäure spezifisch ist; einen Satz gemäß einem der Ansprüche 1 bis 4, der eine Positivkontrollnukleinsäure und eine Sonde, die für die Positivkontrollnukleinsäure spezifisch ist; und zwei oder mehr Reaktionsbehälter umfasst, wobei
der Primersatz, der für die Zielnukleinsäure spezifisch ist, die Sonde, die für die Zielnukleinsäure spezifisch ist, und die Sonde, die für die Positivkontrollnukleinsäure spezifisch ist, in zumindest einem des/r Reaktionsbehälter/s für die Testprobe unter den zwei oder mehr Reaktionsbehältern immobilisiert ist, und wobei der Primersatz, der für die Zielnukleinsäure spezifisch ist, die Sonde, die für die Zielnukleinsäure spezifisch ist, die Positivkontrollnukleinsäure und die Sonde, die für die Positivkontrollnukleinsäure spezifisch ist, in zumindest einem des/r verbleibenden Reaktionsbehälter/s für die Positivkontrollnukleinsäure immobilisiert sind.

6. Kit gemäß Anspruch 5 zur Verwendung beim Nachweis oder der Quantifizierung von zwei unterschiedlichen Arten von Zielnukleinsäuren in einer Testprobe, wobei
das Kit Folgendes umfasst: zwei Arten von Primersätzen, die jeweils für die zwei Arten von Zielnukleinsäuren spezifisch sind; zwei Arten von Sonden, die jeweils für die zwei Arten von Zielnukleinsäuren spezifisch sind; einen Satz, der zwei Arten von Positivkontrollnukleinsäuren umfasst, die jeweils als eine Positivkontrolle für die zwei Arten von Zielnukleinsäuren dienen, und eine oder zwei Arten von Sonden, die für die zwei Arten von Positivkontrollnukleinsäuren spezifisch sind; und zwei oder mehr Reaktionsbehälter, wobei
unter den zwei Arten von Zielnukleinsäuren eine Art von Zielnukleinsäure eine Nukleinsäure eines anderen Gens als des Haushaltsgens ist, und die andere Art von Zielnukleinsäure eine Nukleinsäure eines Haushaltsgens ist,
wobei die zwei Arten von Primersätzen, die zwei Arten von Sonden und die eine oder zwei Arten von Sonden in jedem einzelnen Reaktionsbehälter für die Testprobe immobilisiert sind, und die zwei Arten von Primersätzen, die zwei Arten von Sonden, die zwei Arten von Positivkontrollnukleinsäuren und die eine oder zwei Arten von Sonden in jedem der Reaktionsbehälter für die Positivkontrollnukleinsäuren immobilisiert sind.

7. Kit gemäß Anspruch 5 oder 6, wobei die Polymerase in jedem Reaktionsbehälter ferner immobilisiert ist.

8. Kit gemäß Anspruch 6 oder 7, wobei die zwei oder mehr Reaktionsbehälter drei oder mehr miteinander verbundene Reaktionsbehälter sind,
wobei unter den Reaktionsbehältern ein oder mehrere Reaktionsbehälter ein Reaktionsbehälter für die Testprobe, ein oder mehrere Reaktionsbehälter ein Reaktionsbehälter für die Positivkontrollnukleinsäuren und ein oder mehrere Reaktionsbehälter ein Reaktionsbehälter für eine Negativkontrolle sind, und
wobei die zwei Arten von Primersätzen, die jeweils spezifisch für die zwei Arten von Zielnukleinsäuren sind, die zwei Arten von Sonden, die jeweils spezifisch für die zwei Arten von Zielnukleinsäuren sind, und eine oder zwei Arten von Sonden, die für die zwei Arten von Positivkontrollnukleinsäuren spezifisch sind, im Reaktionsbehälter für eine Negativkontrolle immobilisiert sind.

9. Kit gemäß Anspruch 8, wobei die drei oder mehr miteinander verbundenen Reaktionsbehälter vier oder mehr miteinander verbundene Reaktionsbehälter sind,
wobei zwei oder mehr Reaktionsbehälter Reaktionsbehälter für die Positivkontrollnukleinsäuren unter den Reaktionsbehältern sind,
wobei die Menge der Positivkontrollnukleinsäure, die in den zwei oder mehr Reaktionsbehältern immobilisiert ist, schrittweise differiert, und die Menge der Positivkontrollnukleinsäure in einem Reaktionsbehälter mit der größten Menge der Positivkontrollnukleinsäure unter den zwei oder mehr Reaktionsbehältern die 10-fache oder mehrfache Menge der Positivkontrollnukleinsäure in einem Reaktionsbehälter mit der kleinsten Menge der Positivkontrollnukleinsäure ist.

10. Verfahren zum Nachweis oder zur Quantifizierung einer Zielnukleinsäure in einer Testprobe, während das Vorhandensein oder die Abwesenheit einer Kontamination eines Behälters für die Testprobe mit einer Positivkontrollnukleinsäure bestätigt wird, wobei das Verfahren Folgendes umfasst:
(a) Schritt "a" des Durchführens einer Nukleinsäureamplifikationsreaktion unter Verwendung einer aus der Testprobe erhaltenen Nukleinsäure und eines Primersatzes, der für die Zielnukleinsäure in einem Reaktionsbehälter für die Testprobe spezifisch ist, um ein Amplifikationsprodukt zu erhalten;
(b) Schritt "b" des Durchführens einer Nukleinsäureamplifikationsreaktion unter Verwendung der Positivkontrollnukleinsäure und des Primersatzes, der für die Zielnukleinsäure in einem Reaktionsbehälter für die Positivkontrollnukleinsäure spezifisch ist, um ein Amplifikationsprodukt zu erhalten;
(c) Schritt "c" des Inkontaktbringens des Amplifikationsprodukts, das im Schritt "a" erhalten wurde, mit einer Sonde, die für die Zielnukleinsäure spezifisch ist, und einer Sonde, die für die Positivkontrollnukleinsäure im Reaktionsbehälter für die Testprobe spezifisch ist, und Nachweisen oder Quantifizieren des Vorhandenseins oder der Abwesenheit der Zielnukleinsäure und der Positivkontrollnukleinsäure; und
(d) Schritt "d" des Inkontaktbringens des Amplifikationsprodukts, das im Schritt "b" erhalten wurde, mit der Sonde, die für die Zielnukleinsäure spezifisch ist, und der Sonde, die für die Positivkontrollnukleinsäure im Reaktionsbehälter für die Positivkontrollnukleinsäure spezifisch ist, und Nachweisen oder Quantifizieren des Vorhandenseins oder der Abwesenheit der Zielnukleinsäure und der Positivkontrollnukleinsäure, wobei,
wenn die Positivkontrollnukleinsäure im Schritt "c" nicht nachgewiesen wird, bestätigt werden kann, dass der Behälter für die Testprobe nicht mit der Positivkontrollnukleinsäure kontaminiert ist,
die Positivkontrollnukleinsäure als eine Positivkontrolle der Zielnukleinsäure dient und eine von einer Nukleinsäure ist, die ausgewählt ist aus (I) einer einzelsträngigen Nukleinsäure, die aus einer Nukleotidsequenz mit den folgenden Merkmalen (A) bis (D) besteht, (II) einer einzelsträngigen Nukleinsäure, die aus einer Nukleotidsequenz besteht, die zur Nukleotidsequenz der einzelsträngigen Nukleinsäure (I) komplementär ist, und (III) einer doppelsträngigen Nukleinsäure, die aus der einzelsträngigen Nukleinsäure (I) und der einzelsträngigen Nukleinsäure (II) gebildet wird,
wobei die Positivkontrollnukleinsäure unter Verwendung des Primersatzes, der für die Zielnukleinsäure spezifisch ist, amplifiziert werden kann,
wobei die Nukleotidsequenz der Sonde, die für die Positivkontrollnukleinsäure spezifisch ist, eine Nukleotidsequenz ist, die eine Sequenzidentität von 70 % oder niedriger zur Sequenz eines beliebigen Teils der Genome einer Organismusspezies, von der die Testprobe stammt, und einer Organismusspezies aufweist, von der die Zielnukleinsäure und die Transkripte davon stammen, und die einen Tm-Wert von 65 °C oder niedriger hat; und
wobei die Nukleotidsequenz der Sonde, die für die positive Kontrollnukleinsäure spezifisch ist, eine Nukleotidsequenz ist, die eine Sequenzidentität von 70 % oder niedriger zur Sequenz eines beliebigen Teils der Genome von 20 oder mehr Spezies aufweist, die aus der Gruppe ausgewählt ist, die aus Säugetieren, Bakterien, Pilzen und Viren und den Transkripten davon besteht, und die einen Tm-Wert von 65 °C oder niedriger hat:
(A) wobei die eine Nukleotidsequenz A1 eines 5'-Primers, der für die Zielnukleinsäure spezifisch ist, und eine Nukleotidsequenz A2 umfasst, die zu einem 3'-Primer komplementär ist, der für die Zielnukleinsäure spezifisch ist;
(B) wobei die eine Nukleotidsequenz B1, die zur Sonde komplementär ist, die für die Zielnukleinsäure spezifisch ist, oder eine Nukleotidsequenz B2 umfasst, die zur Nukleotidsequenz B1 komplementär ist;
(C) wobei die eine Nukleotidsequenz C1, die zur Sonde komplementär ist, die für die Positivkontrollnukleinsäure spezifisch ist, oder eine Nukleotidsequenz C2 umfasst, die zur Nukleotidsequenz C1 komplementär ist; und
(D) wobei die Nukleotidsequenz B1 oder B2 und die Nukleotidsequenz C1 oder C2 zwischen den Nukleotidsequenzen A1 und A2 positioniert sind.

11. Verfahren gemäß Anspruch 10 zum Nachweisen oder Quantifizieren von zwei unterschiedlichen Arten von Zielnukleinsäuren in einer Testprobe, während das Vorhandensein oder die Abwesenheit einer Kontamination eines Behälters für die Testprobe mit einer Positivkontrollnukleinsäure bestätigt wird, wobei das Verfahren Folgendes umfasst
Verwenden von zwei Arten von Primersätzen, die jeweils für die zwei Arten von Zielnukleinsäuren spezifisch sind, als den Primersatz, der für die Zielnukleinsäure spezifisch ist,
Verwenden von zwei Arten von Positivkontrollnukleinsäuren als die Positivkontrollnukleinsäure,
Verwenden von zwei Arten von Sonden, die jeweils für die zwei Arten von Zielnukleinsäuren spezifisch sind, als die Sonde, die für die Zielnukleinsäure spezifisch ist,
Verwenden einer oder zweier Arten von Sonden, die für die zwei Arten von Positivkontrollnukleinsäuren spezifisch sind, als die Sonde, die für die Positivkontrollnukleinsäure spezifisch ist, und
Nachweisen oder Quantifizieren des Vorhandenseins oder der Abwesenheit der zwei Arten von Zielnukleinsäuren und der zwei Arten von Positivkontrollnukleinsäuren, wobei,
wenn die Positivkontrollnukleinsäure im Schritt "c" nicht nachgewiesen wird, bestätigt werden kann, dass der Behälter für die Testprobe nicht mit der Positivkontrollnukleinsäure kontaminiert ist, und
unter den zwei Arten von Zielnukleinsäuren eine Art von Zielnukleinsäure eine Nukleinsäure eines anderen Gens als des Haushaltsgens ist, und die andere Art von Zielnukleinsäure eine Nukleinsäure eines Haushaltsgens ist.

## Revendications

1. Trousse destinée à être utilisée pour au moins confirmer la présence ou l'absence de contamination d'un récipient pour un échantillon d'essai avec un acide nucléique témoin positif dans la détection ou la quantification d'un acide nucléique cible dans un échantillon d'essai en utilisant un jeu d'amorces spécifique de l'acide nucléique cible et une sonde spécifique de l'acide nucléique cible, la trousse comprenant un acide nucléique témoin possitif et une sonde pour l'acide nucléique témoin positif,
dans laquelle l'acide nucléique témoin positif sert de témoin positif de l'acide nucléique cible et est l'un quelconque d'un acide nucléique choisi parmi (I) un acide nucléique simple brin constitué par une séquence nucléotidique ayant les caractéristiques suivantes (A) à (D), (II) un acide nucléique simple brin constitué par une séquence nucléotidique complémentaire de la séquence nucléotidique de l'acide nucléique simple brin (I), et (III) un acide nucléique double brin formé à partir de l'acide nucléique simple brin (I) et de l'acide nucléique simple brin (II),
dans laquelle l'acide nucléique témoin positif peut être amplifié en utilisant le jeu d'amorces spécifique de l'acide nucléique cible,
dans laquelle la séquence nucléotidique de la sonde spécifique de l'acide nucléique témoin positif est une séquence nucléotidique qui a 70 % ou moins d'identité de séquence avec la séquence de toute partie des génomes d'une espèce d'organisme de laquelle l'échantillon d'essai est dérivé et d'une espèce d'organisme de laquelle l'acide nucléique cible est dérivé, et des produits de transcription de ceux-ci, et a une valeur Tm de 65 °C ou moins ; et
dans laquelle la séquence nucléotidique de la sonde spécifique de l'acide nucléique témoin positif est une séquence nucléotidique qui a 70 % ou moins d'identité de séquence avec la séquence de toute partie des génomes de 20 espèces ou plus choisies parmi le groupe constitué par des mammifères, des bactéries, des champignons et des virus, et des produits de transcription de ceux-ci, et a une valeur Tm de 65 °C ou moins :
(A) comprenant une séquence nucléotidique A1 d'une amorce en 5' spécifique de l'acide nucléique cible et une séquence nucléotidique A2 complémentaire d'une amorce en 3' spécifique de l'acide nucléique cible ;
(B) comprenant une séquence nucléotidique B1 complémentaire de la sonde spécifique de l'acide nucléique cible, ou une séquence nucléotidique B2 complémentaire de la séquence nucléotidique B1 ;
(C) comprenant une séquence nucléotidique C1 complémentaire de la sonde spécifique de l'acide nucléique témoin positif, ou une séquence nucléotidique C2 complémentaire de la séquence nucléotidique C1 ; et
(D) la séquence nucléotidique B1 ou B2 et la séquence nucléotidique C1 ou C2 sont positionnées entre les séquences nucléotidiques A1 et A2.

2. Trousse selon la revendication 1, dans laquelle la séquence nucléotidique (I) dans la séquence nucléotidique de l'acide nucléique témoin positif est
(I') une séquence nucléotidique dans laquelle la séquence nucléotidique C1 ou C2 est insérée dans une partie autre que les séquences nucléotidiques A1 et A2 et la séquence nucléotidique B1 ou B2 dans un acide nucléique simple brin ayant les séquences nucléotidiques A1 et A2 et la séquence nucléotidique B1 ou B2 dans une séquence nucléotidique dérivée de la séquence nucléotidique de l'acide nucléique cible, ou
une séquence nucléotidique dans laquelle une ou plusieurs séquences nucléotidiques d'une partie autre que les séquences nucléotidiques A1 et A2 et la séquence nucléotidique B1 ou B2 sont substituées avec la séquence nucléotidique C1 ou C2 dans un acide nucléique simple brin ayant les séquences nucléotidiques A1 et A2 et la séquence nucléotidique B1 ou B2 dans une séquence nucléotidique dérivée de la séquence nucléotidique de l'acide nucléique cible.

3. Trousse selon la revendication 1 ou 2, dans laquelle le nombre de nucléotides des séquences nucléotidiques A1 et A2 est compris dans la plage de 15 à 50, respectivement, le nombre de nucléotides des séquences nucléotidiques B1 et B2 est compris dans la plage de 15 à 100, respectivement, le nombre de nucléotides des séquences nucléotidiques C1 et C2 est compris dans la plage de 15 à 100, respectivement, et le nombre de nucléotides de la sonde spécifique de l'acide nucléique témoin positif est compris dans la plage de 15 à 100.

4. Trousse selon l'une quelconque des revendications 1 à 3, dans laquelle la séquence nucléotidique de la sonde spécifique de l'acide nucléique témoin positif est une séquence nucléotidique ayant une liaison de deux séquences nucléotidiques ou plus, qui est choisie parmi le groupe constitué de séquences nucléotidiques répétées ayant 4 à 8 nucléotides obtenues en répétant deux à quatre fois l'une quelconque des séquences nucléotidiques suivantes ayant 2 nucléotides :
AT, AG, AC, TA, TG, TC, GA, GT, GC, CA, CT, et CG.

5. Trousse destinée à être utilisée dans la détection ou la quantification d'un acide nucléique cible dans un échantillon d'essai, la trousse comprenant : un jeu d'amorces spécifique de l'acide nucléique cible ; une sonde spécifique de l'acide nucléique cible ; un jeu selon l'une quelconque des revendications 1 à 4 comprenant un acide nucléique témoin positif et une sonde spécifique de l'acide nucléique témoin positif ; et deux récipients de réaction ou plus, dans laquelle
le jeu d'amorces spécifique de l'acide nucléique cible, la sonde spécifique de l'acide nucléique cible, et la sonde spécifique de l'acide nucléique témoin positif sont immobilisés dans au moins l'un du ou des récipients de réaction pour l'échantillon d'essai parmi les deux récipients de réaction ou plus, et le jeu d'amorces spécifique de l'acide nucléique cible, la sonde spécifique de l'acide nucléique cible, l'acide nucléique témoin positif, et la sonde spécifique de l'acide nucléique témoin positif sont immobilisés dans au moins l'un du ou des récipients de réaction restants pour l'acide nucléique témoin positif.

6. Trousse selon la revendication 5 destinée à être utilisée dans la détection ou la quantification de deux types différents d'acides nucléiques cibles dans un échantillon d'essai, dans laquelle
la trousse comprend : deux types de jeux d'amorces chacun spécifiques des deux types d'acides nucléiques cibles ; deux types de sondes chacune spécifiques des deux types d'acides nucléiques cibles ; un jeu comprenant deux types d'acides nucléiques témoins positifs chacun servant de témoin positif pour les deux types d'acides nucléiques cibles, et un ou deux types de sondes spécifique des deux types d'acides nucléiques témoins positifs ; et deux récipients de réaction ou plus, dans laquelle
parmi les deux types d'acides nucléiques cibles, un type d'acide nucléique cible est un acide nucléique d'un gène autre que le gène domestique, et l'autre type d'acide nucléique cible est un acide nucléique d'un gène domestique,
les deux types de jeux d'amorces, les deux types de sondes, et les un ou deux types de sondes sont immobilisés dans chaque récipient de réaction individuel pour l'échantillon d'essai, et les deux types de jeux d'amorces, les deux types de sondes, les deux types d'acides nucléiques témoins positifs, et les un ou deux types de sondes sont immobilisés dans chaque récipient de réaction pour les acides nucléiques témoins positifs.

7. Trousse selon la revendication 5 ou 6, dans laquelle la polymérase est immobilisée en outre dans chaque récipient de réaction.

8. Trousse selon la revendication 6 ou 7, dans laquelle les deux récipients de réaction ou plus sont trois récipients de réaction ou plus connectés les uns aux autres,
parmi les récipients de réaction, un ou plusieurs récipients de réaction sont un récipient de réaction pour l'échantillon d'essai, un ou plusieurs récipients de réaction sont un récipient de réaction pour les acides nucléiques témoins positifs, et un ou plusieurs récipients de réaction sont un récipient de réaction pour un témoin négatif, et
dans lequel les deux types de jeux d'amorces chacun spécifiques des deux types d'acides nucléiques cibles, les deux types de sondes chacune spécifiques des deux types d'acides nucléiques cibles, et un ou deux types de sondes spécifique des deux types d'acides nucléiques témoins positifs sont immobilisés dans le récipient de réaction pour un témoin négatif.

9. Trousse selon la revendication 8, dans laquelle les trois récipients de réaction ou plus connectés les uns aux autres sont quatre récipients de réaction ou plus connectés les uns aux autres,
deux récipients de réaction ou plus sont des récipients de réaction pour les acides nucléiques témoins positifs parmi les récipients de réaction,
la quantité de l'acide nucléique témoin positif immobilisé dans les deux récipients de réaction ou plus diffère progressivement, et la quantité de l'acide nucléique témoin positif dans un récipient de réaction ayant la plus grande quantité de l'acide nucléique témoin positif parmi les deux récipients de réaction ou plus représente 10 fois ou plus la quantité de l'acide nucléique témoin positif dans un récipient de réaction ayant la plus petite quantité de l'acide nucléique témoin positif.

10. Procédé destiné à détecter ou à quantifier un acide nucléique cible dans un échantillon d'essai tout en confirmant la présence ou l'absence de contamination d'un récipient pour l'échantillon d'essai avec un acide nucléique témoin positif, le procédé comprenant :
(a) l'étape « a » de réalisation d'une réaction d'amplification d'acide nucléique en utilisant un acide nucléique obtenu à partir de l'échantillon d'essai et un jeu d'amorces spécifique de l'acide nucléique cible dans un récipient de réaction pour l'échantillon d'essai pour obtenir un produit d'amplification ;
(b) l'étape «b» de réalisation d'une réaction d'amplification d'acide nucléique en utilisant l'acide nucléique témoin positif et le jeu d'amorces spécifique de l'acide nucléique cible dans un récipient de réaction pour l'acide nucléique témoin positif pour obtenir un produit d'amplification ;
(c) l'étape « c » de mise en contact du produit d'amplification obtenu dans l'étape « a » avec une sonde spécifique de l'acide nucléique cible et une sonde spécifique de l'acide nucléique témoin positif dans le récipient de réaction pour l'échantillon d'essai, et de détection ou de quantification de la présence ou de l'absence de l'acide nucléique cible et de l'acide nucléique témoin positif ; et
(d) l'étape « d » de mise en contact du produit d'amplification obtenu dans l'étape « b » avec la sonde spécifique de l'acide nucléique cible et la sonde spécifique de l'acide nucléique témoin positif dans le récipient de réaction pour l'acide nucléique témoin positif, et de détection ou de quantification de la présence ou de l'absence de l'acide nucléique cible et de l'acide nucléique témoin positif, dans lequel
quand l'acide nucléique témoin positif n'est pas détecté dans l'étape « c », il peut être confirmé que le contenant pour l'échantillon d'essai n'est pas contaminé avec l'acide nucléique témoin positif,
l'acide nucléique témoin positif sert de témoin positif de l'acide nucléique cible et est l'un quelconque d'un acide nucléique choisi parmi (I) un acide nucléique simple brin constitué par une séquence nucléotidique ayant les caractéristiques suivantes (A) à (D), (II) un acide nucléique simple brin constitué par une séquence nucléotidique complémentaire de la séquence nucléotidique de l'acide nucléique simple brin (I), et (III) un acide nucléique double brin formé à partir de l'acide nucléique simple brin (I) et de l'acide nucléique simple brin (II),
dans lequel l'acide nucléique témoin positif peut être amplifié en utilisant le jeu d'amorces spécifique de l'acide nucléique cible,
dans lequel la séquence nucléotidique de la sonde spécifique de l'acide nucléique témoin positif est une séquence nucléotidique qui a 70 % ou moins d'identité de séquence avec la séquence de toute partie des génomes d'une espèce d'organisme de laquelle l'échantillon d'essai est dérivé et d'une espèce d'organisme de laquelle l'acide nucléique cible est dérivé, et des produits de transcription de ceux-ci, et a une valeur Tm de 65 °C ou moins ; et
dans lequel la séquence nucléotidique de la sonde spécifique de l'acide nucléique témoin positif est une séquence nucléotidique qui a 70 % ou moins d'identité de séquence avec la séquence de toute partie des génomes de 20 espèces ou plus choisies parmi le groupe constitué par des mammifères, des bactéries, des champignons et des virus, et des produits de transcription de ceux-ci, et a une valeur Tm de 65 °C ou moins :
(A) comprenant une séquence nucléotidique A1 d'une amorce en 5' spécifique de l'acide nucléique cible et une séquence nucléotidique A2 complémentaire d'une amorce en 3' spécifique de l'acide nucléique cible ;
(B) comprenant une séquence nucléotidique B1 complémentaire de la sonde spécifique de l'acide nucléique cible, ou une séquence nucléotidique B2 complémentaire de la séquence nucléotidique B1 ;
(C) comprenant une séquence nucléotidique C1 complémentaire de la sonde spécifique de l'acide nucléique témoin positif, ou une séquence nucléotidique C2 complémentaire de la séquence nucléotidique C1 ; et
(D) la séquence nucléotidique B1 ou B2 et la séquence nucléotidique C1 ou C2 sont positionnées entre les séquences nucléotidiques A1 et A2.

11. Procédé selon la revendication 10 destiné à détecter ou à quantifier deux types différents d'acides nucléiques cibles dans un échantillon d'essai tout en confirmant la présence ou l'absence de contamination d'un récipient pour l'échantillon d'essai avec un acide nucléique témoin positif, le procédé comprenant
l'utilisation de deux types de jeux d'amorces chacun spécifiques des deux types d'acides nucléiques cibles comme le jeu d'amorces spécifique de l'acide nucléique cible,
l'utilisation de deux types d'acides nucléiques témoins positifs comme l'acide nucléique témoin positif,
l'utilisation de deux types de sondes chacune spécifiques des deux types d'acides nucléiques cibles comme la sonde spécifique de l'acide nucléique cible,
l'utilisation d'un ou de deux types de sondes spécifiques des deux types d'acides nucléiques témoins positifs comme la sonde spécifique de l'acide nucléique témoin positif, et
la détection ou la quantification de la présence ou de l'absence des deux types d'acides nucléiques cibles et des deux types d'acides nucléiques témoins positifs, dans lequel
quand l'acide nucléique témoin positif n'est pas détecté dans l'étape « c », il peut être confirmé que le récipient pour l'échantillon d'essai n'est pas contaminé avec l'acide nucléique témoin positif, et
parmi les deux types d'acides nucléiques cibles, un type d'acide nucléique cible est un acide nucléique d'un gène autre que le gène domestique, et l'autre type d'acide nucléique cible est un acide nucléique d'un gène domestique.


### SEQUENCE LISTING

<110> National University Corporation Tokyo Medical and Dental University NIHON TECHNO SERVICE CO., LTD.
<120> Method of detection and quantitation of a target nucleic acid in a
   test sample utilizing a novel positive control nucleic acid
<130> P14-003P
<150> JP 2014-212496
   <151> 2014-10-17
<160> 17
<170> PatentIn version 3.1
<210> 1
   <211> 28
   <212> DNA
   <213> Artificial
<220>
   <223> probe 1 specific for positive control nucleic acid
<220>
   <221> misc_feature
   <223> Inventor: Shimizu, Norio Inventor: Watanabe, Ken Inventor: Tomaru, Yasuhiro
<400> 1
   cacaatatac acgcgctata tctcacac 28
<210> 2
   <211> 28
   <212> DNA
   <213> Artificial
<220>
   <223> probe 2 specific for positive control nucleic acid
<400> 2
   atatgagaac actgtgtctc tatagcgc 28
<210> 3
   <211> 28
   <212> DNA
   <213> Artificial
<220>
   <223> probe 3 specific for positive control nucleic acid
<400> 3
   atatcacaac actatatctc acacgcgc 28
<210> 4
   <211> 3048
   <212> DNA
   <213> Epstein-Barr virus
<400> 4
   ttagaatggt ggccgggctg taaaattctg gaggacggag agggcggccc cggagttgtt 60
   atcaaagagg cactggagga tgttggccgc tccttggagc agcttgtcga aataatgatc 120
   cacggccacg ggaacgccgt gccgctcggc gtaggccggg tcctcggcca tctccgtctt 180
   tctcgccccc ttcactcccc ccttgggctc cacaaagacg tactggatgc ggtcgtggat 240
   ctggggcagt tcctcgttgc gctcgacgaa cttctggtag acggccaggt gaggcatctg 300
   ggtgctcttg taggctgaga gcttgcggct gagctccgtt gaaaagcaga gctcccccat 360
   ggggaccctg ccttcacgga ggtctgtgta ggcctggttt aggatgtcaa tgacgggcaa 420
   aaagcccaca ggtagccctt gtgtaaatga ctcttggaag ggccggtggg agaggaggct 480
   ggccgcctcc tttacccggg catccgccag caccaggtcg agcacgcgcc ggcagcgtgt 540
   ctgcacaaac ttgcaggccg tcttccggac gagctccacc cccttcatca gggtcttgcc 600
   gtccgtcagc acccccacat atctcttctt tgtaatcagc atcaggcagg agaaggtctt 660
   ctcggcctcc agggagatgg gggccacaaa caggctccgg gtggtgtggg cggccagggc 720
   atcggcaaag cgcagggtct cgctctctga aaacccccgg cactcgataa acagcgagtc 780
   cgtgtccccg tagatgactc gaagctggcc ctcggggttg aggggcgccc aggcgtccgg 840
   ggagggggcc agggcctgca ggttggcggg gctcagggcc tccacgaagg ccttggcccg 900
   ctccaacatc gtgcggccct gcagcgtcac cgtctcggcg atggagaggc agggaaagag 960
   gccgttggcc accccggtga agccgtagac ggcgttgcac gtgcacttga tggccagctg 1020
   ctgcttgtcg aggatggtcc tttggcgcgg atcctcgcag gccgccagca gcttcttgat 1080
   ggccttgcgc ttggccagcc aggaggtcaa cagactagcc aagaaggact cgtgcacgtg 1140
   cttctttaca aagtggtaga cgccccccgt gagcctgaag gactcatagt cttctcccgg 1200
   gcgcaggccg gctagcctgt gctcttctcc cggcgttatc atggtagaat aacagagatt 1260
   atgagcctga atgatgctcg ggtagaggct ggcaaagtcc accaccagaa ccggggagtt 1320
   gtagaatccg gacaggggct ggatgacggt ggccccctgg tagccgtccc ggtcagaggc 1380
   cgagggcatg ggcaggataa agttttcctt ttgggcggcc gccaggaggc aggagaacac 1440
   gcggatctgc tgcccatcgt ccagcacccg cctgcagggg atgtgagcga tcttggcaat 1500
   ctctgccacc tccacgtgga tcacgaaatg gtttagcaga tccatgacca gggccgagtc 1560
   ctgcacgcag tacatgccga gccgcctgcg cccctcgggg cccgctgcaa agaggcgagg 1620
   aatctccttg taatgcacat cctccttctt ggcccccagt aggtgcctgg ctactgtgtc 1680
   cagcttgtag tctgagaggc tgagcttgtc ccggcacacg gcgtacatgt cgatggggat 1740
   gaggccggtg atgcggacct tggtgttggc ccgcaagaag cccttgcccg catcatgggg 1800
   tcgcctgacc tcgcagacgc ccccagccct aattttgccc agagaggctg ggttgatgct 1860
   gtagatgtgc ctggctctgt ccagaatgta gggccagtca aagttggcca cgttgtagcc 1920
   ggtcacaatc tccacgctga ggtctctgat gagctggaag aaggcgtaga gcatgtccag 1980
   ctccgatggg aactcgtaga cctcaacccc ctctatgtct tcgcaggtgc ccagcgtcag 2040
   caggatgcgc ctatagcgcc cggcctcctc ccctgtcgac cagaggacgc aggatatctg 2100
   caggatcagg tcagcctcgt tggtggccgt ggggaagccc tcctccccca gacactcgat 2160
   atcgaaggcc agggcctggt aggagggcca ggagctgtct tcacgccgga ccgagaggtc 2220
   gcccacctca cagtcgtact cgagctcggc gtacgagtcc cggtgctgga ggcgggggat 2280
   ggcgcggcgg cagctgtacc agccaaaggt gacaaagtca ttgtccagga caaagcggcg 2340
   cgtggcatcc acgttggcct caaagatccg acacccgtgc ttgtcttgca gccacgtggc 2400
   cacgtgacac acactgttgg gatgggagag ggtgatcttg tggtagtcgc cggcatggtt 2460
   gccgtagccc ataatggaac ggcgcgtgac cttctccacc gagacccggc agggggtcct 2520
   gcggtcgaag gtgctggcct tgagggcgct gaggactgca aactccacgt ccagaccctg 2580
   aggcgcgctg gcgtagaagt aggcctgctg cccaaacacg ttcacacaca cgctggcccc 2640
   atcggccttg cgccggccca gtagcttgat gacgatgcca catggcacca catacccctg 2700
   tttatccgat ggaatgacgg cgcatttctc gtgcgtgtac accgtctcga gtatgtcgta 2760
   gacatggaag tccagagggc ttccgtgggt gtctgcctcc ggccttgccg tgccctcttg 2820
   ggcacgctgg cgccaccaca tgccctttcc atcctcgtca ccccccacca ccgtcaggga 2880
   gtcttggtag aagcacaggg ggggctgagg cccccgcaca tccaccaccc ctgcggcgcc 2940
   tggtgtctgg aaacacttgg gaatgagacg caggtactcc ttgtcaggct ttttcagaag 3000
   gcctttatta ggtcttagga aagggttata gaagagtccc ccagacat 3048
<210> 5
   <211> 140
   <212> DNA
   <213> Artificial
<220>
   <223> Target nucleic acid EBV
<400> 5
   tagggccagt caaagttggc cacgttgtag ccggtcacaa tctccacgct gaggtctctg 60
   atgagctgga agaaggcgta gagcatgtcc agctccgatg ggaactcgta gacctcaacc 120
   ccctctatgt cttcgcaggt 140
<210> 6
   <211> 168
   <212> DNA
   <213> Artificial
<220>
   <223> Positive control nucleic acid used for EBV
<400> 6
   tagggccagt caaagttggc cacgttgtag ccggtcacaa tctccacgct gaggtctctg      60
   atgagctgga agaaggcgta gagcatgtcc agctccgatg cgctatagag acacagtgtt     120
   ctcatatggg aactcgtaga cctcaacccc ctctatgtct tcgcaggt                  168
<210> 7
   <211> 18
   <212> DNA
   <213> Artificial
<220>
   <223> EBV-F primer
<400> 7
   tagggccagt caaagttg 18
<210> 8
   <211> 18
   <212> DNA
   <213> Artificial
<220>
   <223> EBV-R primer
<400> 8
   acctgcgaag acatagag 18
<210> 9
   <211> 20
   <212> DNA
   <213> Artificial
<220>
   <223> EBV-probe
<400> 9
   cggtcacaat ctccacgctg 20
<210> 10
   <211> 81
   <212> DNA
   <213> Artificial
<220>
   <223> Target nucleic acid TBP
<400> 10
   ggcaccactc cactgtatcc ctcccccatg actcccatga cccccatcac tcctgccacg 60
   ccagcttcgg agagttctgg g 81
<210> 11
   <211> 20
   <212> DNA
   <213> Artificial
<220>
   <223> TBP-F primer
<400> 11
   gcaccactcc actgtatccc 20
<210> 12
   <211> 20
   <212> DNA
   <213> Artificial
<220>
   <223> TBP-R primer
<400> 12
   cccagaactc tccgaagctg 20
<210> 13
   <211> 109
   <212> DNA
   <213> Artificial
<220>
   <223> Positive control nucleic acid used for TBP
<400> 13
   ggcaccactc cactgtatcc ctcccccatg cgctatagag acacagtgtt ctcatatgac 60
   tcccatgacc cccatcactc ctgccacgcc agcttcggag agttctggg 109
<210> 14
   <211> 22
   <212> DNA
   <213> Artificial
<220>
   <223> TBP-probe
<400> 14
   acccccatca ctcctgccac gc 22
<210> 15
   <211> 24
   <212> DNA
   <213> Artificial
<220>
   <223> IC-probe
<400> 15
   cacatgtgat atgcgcagag tctc 24
<210> 16
   <211> 164
   <212> DNA
   <213> Artificial
<220>
   <223> positive control
<400> 16
   tagggccagt caaagttggc cacgttgtag ccggtcacaa tctccacgct gaggtctctg     60
   atgagctgga agaaggcgta gagcatgtcc agctccgatg agactctgcg catatcacat    120
   gtggggaact cgtagacctc aaccccctct atgtcttcgc aggt                     164
<210> 17
   <211> 105
   <212> DNA
   <213> Artificial
<220>
   <223> positive control
<400> 17
   ggcaccactc cactgtatcc ctcccccatg agactctgcg catatcacat gtggactccc     60
   atgaccccca tcactcctgc cacgccagct tcggagagtt ctggg                    105